# EUROPEAN PATENT SPECIFICATION

(11) **EP 4 034 880 B1**
(45) Date of publication and mention of the grant of the patent: **09.09.2026**
(21) Application number: 20775666.9
(22) Date of filing: 25.09.2020
(51) Int. Cl.: G01N 33/569, G01N 33/92

(54) **A NOVEL COMPLEX FORMED BETWEEN THE FLAVIVIRAL NON-STRUCTURAL NS1 PROTEIN AND PLASMA LIPOPROTEINS**
NEUER KOMPLEX, DER ZWISCHEN DEN FLAVIVIRALEN NICHT-STRUKTURELLEN NS1-PROTEIN- UND PLASMA-LIPOPROTEINEN GEBILDET WIRD
NOUVEAU COMPLEXE FORMÉ ENTRE LA PROTÉINE NS1 NON STRUCTURALE FLAVIVIRALE ET LES LIPOPROTÉINES DE PLASMA

(30) Priority: 25.09.2019 EP 19306200
(43) Date of publication of application: 03.08.2022
(73) Proprietor: Institut Pasteur, 75724 Paris Cédex 15 (FR); CENTRE NATIONAL DE LA RECHERCHE SCIENTIFIQUE (CNRS), 75016 Paris (FR); Institut Pasteur Du Cambodge, Phnom Penh (KH)
(72) Inventor: FLAMAND, Marie, 91190 Gif sur Yvette (FR); PARK, Kyu-Ho Paul, 93150 Le Blanc Mesnil (FR); BENFRID, Souheyla, 75012 Paris (FR); TAMIETTI, Carole, 75724 Paris Cedex 15 (FR); VOSS, James, 75724 Paris Cedex 15 (FR); COULIBALY, Fasséli, Victoria, Victoria 3151 (AU); DUONG, Veasna, Phnom Penh (KH); DUSSART, Philippe, Phnom Penh (KH); SAKUNTABHAI, Anavaj, 75011 Paris (FR); GIAI GIANETTO, Quentin, 75724 Paris Cedex 15 (FR); MATONDO, Mariette, 78630 Orgeval (FR); DELLAROLE, Mariano, 92130 Issy Les Moulineaux (FR); BONTEMS, François, 75014 Paris (FR); REY, Félix, 75724 Paris Cedex 15 (FR)
(74) Representative: Casalonga
(86) International application number: PCT/EP2020/077014
(87) International publication number: WO 2021/058809

(56) References cited:
- WO-A1-2017/144173
- WO-A2-2015/196192
- TAKASUKE FUKUHARA ET AL: "Host-derived apolipoproteins play comparable roles with viral secretory proteins E rns and NS1 in the infectious particle formation of Flaviviridae", 23 June 2017 (2017-06-23), XP055678269, Retrieved from the Internet <URL:https://journals.plos.org/plospathogens/article/file?id=10.1371/journal.ppat.1006475&type=printable> [retrieved on 20200320]
- NAGESWAR REDDY MANCHALA ET AL: "Proteomic analysis reveals the enhancement of human serum apolipoprotein A-1(APO A-1) in individuals infected with multiple dengue virus serotypes", TROPICAL MEDICINE AND INTERNATIONAL HEALTH., vol. 22, no. 10, 18 August 2017 (2017-08-18), GB, pages 1334 - 1342, XP055678370, ISSN: 1360-2276, DOI: 10.1111/tmi.12931
- YUJIA LI ET AL: "Human Apolipoprotein A-I Is Associated with Dengue Virus and Enhances Virus Infection through SR-BI", PLOS ONE, vol. 8, no. 7, 24 July 2013 (2013-07-24), pages e70390, XP055678362, DOI: 10.1371/journal.pone.0070390
- HONGWEI LI ET AL: "FUNCTIONAL ANALYSIS OF DENGUE VIRUS NONSTRUCTURAL PROTEIN 1", 1 August 2014 (2014-08-01), XP055678254, Retrieved from the Internet <URL:https://scholarspace.manoa.hawaii.edu/bitstream/10125/100411/1/Li_Yujia_r.pdf> [retrieved on 20200320]

## Description

### FIELD OF THE INVENTION

The invention relates to diagnostic, monitoring and prognostic methods of a flaviviral infection, and more particularly of a Dengue virus infection. The invention relates to methods involving detecting and/or measuring the level of complexes formed by the flaviviral non-structural glycoprotein NS1 and plasma high-density lipoprotein particles (HDL) as set out in the appended claims.

### BACKGROUND OF THE INVENTION

Dengue is a global mosquito-borne viral disease that threatens 3.9 billion people in 128 countries, and represents a leading cause of hospitalization and death in tropical and subtropical areas of the world. Dengue virus is transmitted by female mosquitoes mainly of the species *Aedes aegypti* and, to a lesser extent, *Ae. Albopictus.* Dengue is caused by a virus of the Flaviviridae family and there are 4 distinct, but closely related, serotypes of the virus that cause dengue (DEN-1, DEN-2, DEN-3 and DEN-4). The four dengue virus serotypes are estimated to infect 390 million individuals per year (95% credible interval 284-528 million), of which 96 million (67-136 million) manifest clinically (with any severity of disease) (Bhatt et al. 2013).

The clinical characteristics of dengue are wide-ranging, from mild conditions to life-threatening symptoms. More rarely, atypical occurrences of dengue virus infection may involve fulminant hepatitis, cardiomyopathy, acute renal failure, and encephalopathy. The course of the disease can be divided in three phases: (i) the acute febrile phase lasting 2 to 7 days with non-specific clinical signs and possible mild hemorrhagic symptoms (petechial and mucosal bleeding), (ii) the critical phase generally occurring at the time of defervescence, during which complications may appear in a small proportion of patients, including severe hemorrhage, plasma leakage with shock, and organ impairment, and (iii) the recovery phase. Early and appropriate management of dengue cases with severe symptoms is a key step in reducing mortality.

The WHO proposed the first guidelines on dengue with a clinical classification in 1974, which was revised in 1997. This WHO 1997 classification scheme, which comprises three categories - dengue fever (DF), dengue hemorrhagic fever (DHF), and dengue shock syndrome (DSS) - is based on prerequisite clinical and/or biological signs to correctly classify the degree of severity of the disease. Moreover, as dengue case attack rates increased and disease became widespread in Asia and the Americas, many clinicians confronting the full spectrum of dengue encountered difficulties in applying the WHO 1997 case definitions for triage and clinical management. Follow up prospective clinical study conducted across countries in Latin America and Asia aimed to improve dengue case management, the WHO launched new case management guidelines in 2009 (WHO 2009), which instead of being longitudinal (previous step required before passing to next step), provided a cross-sectional classification of dengue cases that established criteria to identify dengue cases with warning signs or severe dengue (SVD).

DENV is a small, enveloped, positive single-stranded RNA that encodes three structural (envelope, membrane and capsid) and seven non-structural (NS) proteins (NS1, NS2a, NS2b, NS3, NS4a, NS4b and NS5) (Guzman et al. 2010). The nonstructural protein 1 (NS1) participates to viral replication in DENV-infected cells and can be shed in large amounts in the bloodstream of patients experiencing the various clinical grades of dengue disease (Alcon-LePoder et al. 2006). NS1 was shown to bind complement and coagulation factors and to trigger the production of antibodies cross-reactive with surface antigens of platelets and endothelial cells and has thus been proposed to play a role in the development of thrombocytopenia and hemorrhage in dengue disease (Rastogi et al. 2016). More recently, the secreted form of NS1 was shown to promote endothelium permeability and vascular leakage *in vitro* and *in vivo,* and to critically contribute to the inflammatory cytokine storm observed in severe dengue cases (de Silva et al. 2018).

Currently, there is a commercially available vaccine for dengue virus that has shown adverse effects and no specific therapy. In the absence of immunization, the monitoring of dengue virus outbreaks and serological mapping become critically important to the control and containment of infection. As clinical manifestations for dengue virus infections are quite unspecific, it is difficult to affirm diagnosis without laboratory testing. Programs have been set up by WHO to actively monitor vector insects and cases of fever, as well as to perform serological and virological screening of individuals suspected of being infected with dengue virus. Thus, the development of diagnostic assays for dengue infection is critically important.

Early diagnosis is essential for proper timely treatment of the patient, access to proper medical care lowering fatality rates below 1%. The currently available tests for dengue include RT-PCR for viral RNA and immunological tests for dengue-specific antibody or viral proteins. However, many of these tests have significant disadvantages. For example, RT-PCR for viral RNA requires expensive laboratory equipment and trained personnel, which makes it hard to use on a large scale or in rural areas. Some dengue-specific enzyme linked immunosorbent assays (ELISAs) can detect IgM or IgG that appear later during the course of infection, however diagnosis as early as day two of infection is preferable.

A comparative analysis of four diagnostic methods for dengue infection, namely virus isolation, viral RNA detection, dengue specific IgM detection and NS1 antigen detection revealed that NS1 antigen detection had the highest sensitivity rate compared to the other three methods (Kumarasamy et al. 2007). Alcon *et al.* 2002 have described an ELISA for NS1 detection and demonstrated that NS1 is present at high levels in patient sera during primary and secondary infection. NS1 is detectable during the whole clinical phase of illness and can be detected in the first few days of infection (as early as the first day of fever). Falconar and Young, 1991 have described the production of dimer-specific and dengue virus group cross-reactive mouse monoclonal antibodies to DEN2 virus NS1 and the use of certain of these antibodies in an ELISA for NS1 (Young et al. 2000). High levels of NS1 were found in acute phase sera, but not in convalescent phase sera, from some of the patients with serologically confirmed DEN2 virus secondary infection. PCT Patent Application WO 00/75665 describes a method for detecting NS1 protein in the hexameric form, and the selection of antibodies directed against the NS1 secreted hexameric form, together with the use of such antibodies in the early detection of flavivirus infection.

PCT application WO 2015/196192 discloses a method for detecting dengue virus infection using antibodies which specifically bind to a defined epitope sequence of dengue virus (DENV) non-structural protein 1 (NS1); a heating step effective to irreversibly denature the 3-D structure of DENV NS1 in a patient sample resulted in at least three-fold increase in OD450 in the capture ELISA, allowing the detection of DENV NS1 below the lower serum concentration of 50 µg/ml found in acute-dengue patients.

Hongwei Li et al.: "Functional Analysis of Dengue Virus Nonstructural protein 1", 1 August 2014 investigate protein candidate that is interacted with Dengue virus (DV) particles and DV non-structural protein 1 (NS1) in vitro in cell culture supernatant, by co-immunoprecipitation assay using recombinant NS1-FLAG and/or ApoA1-FLAG. Analysis of co-immunoprecipitates by SDS-PAGE followed by western-blot using anti-FLAG, anti-NS1, anti-ApoA1 and/or anti-E antibodies indicate that Apolipoprotein A1 (ApoA1) can interact with both Dengue virus NS1 and Dengue virus particles.

PCT application WO 2017/144173 discloses a method for detecting anti-NS1 antibodies in a biological sample from a flavivirus infected subject using a complex of recombinant NS1 antigen and Apoliprotein A1, which is more stable in solution than the NS1 polypeptide by itself and enhances the diagnostic reliability and stability of the NS1 antigen.

The problem underlying the invention therefore is to provide new markers of dengue infection that have an added diagnostic and prognostic value compared to the commonly used NS1 marker, and more particularly to allow for better medical care of patients at risk to develop a severe dengue, and to decrease mortality rates.

The problem is solved by the current invention as specified in the claims.

### DESCRIPTION OF THE INVENTION

The inventors found that Dengue virus NS1, secreted as an atypical lipoprotein particle by infected cells, binds with a strong affinity to serum high-density lipoprotein particles (HDL), a major player of vascular homeostasis, inflammation and thrombosis, and to a lower extent to low density lipoproteins particles (LDL). The NS1-HDL complex, and not the NS1 protein alone, induces cytokine production in primary human macrophages suggesting that this complex represents a previously unreported active form of the viral protein. In parallel, the inventors developed three ELISA based on the NS1-ApoA1, NS1-ApoB and NS1-ApoE detection in DENV-infected patients to assess the concentration of complexes formed between NS1 and HDL, NS1 and LDL or between NS1 and a larger panel of lipoprotein particles (possibly HDL, IDL, LDL, VLDL and chylomicrons), respectively. The NS1-ApoA1 complex is detected in the majority of the acute phase blood samples and appears to be inversely correlated with the number of days of fever. In contrast, the concentration of the NS1-ApoE complex increases over time during the 12-days observation period. A biostatistical analysis of the different virological and clinical markers revealed that the concentrations of the NS1-HDL complexes are inversely correlated to the degree of severity of the disease and in particular to plasma leakage intensity. The NS1-HDL complex thus represents a novel diagnostic marker that is more pertinent than the NS1 protein itself, and with an added prognostic value that remains to be more thoroughly assessed in a larger clinical study.

The invention is as set out in the appended claims.

The invention concerns an *in vitro* method for the diagnostic, prognostic or monitoring of a flaviviral infection or associated disease, comprising detecting the presence or level of a complex formed by a flaviviral non-structural protein 1 (NS1) and plasma high-density lipoprotein (HDL) particles in a biological sample obtained from a subject.

The complex may be detected by any appropriate means, using standard techniques, such as chemical, physical or other techniques. In some embodiments, the complex is detected using immunochemical techniques.

For the purpose of the present invention, the term "non-structural protein 1" is used interchangeably with "non-structural glycoprotein 1", and "NS1", and encompass the native protein obtained from the culture supernatant of mammalian cells infected with a flavivirus or transformed using an expression system comprising the gene of the NS1 protein of said flavivirus, and purified. NS1 is in hexameric form, monomeric form, or dimeric form.

Herein disclosed is an *in vitro* method for quantitating a complex formed by the flaviviral non-structural protein 1 (NS1) and endogenous lipoprotein particles, in particular plasma lipoprotein particles, in a biological sample obtained from a subject, comprising:
a. Contacting the biological sample with an antibody specific for the flaviviral NS1 and/or an antibody specific for endogenous lipoproteins, in particular plasma lipoproteins, to form an immunoreaction product;
b. detecting the presence of the immunoreaction product, and
c. quantitating the complex formed by NS1 and endogenous lipoprotein particles, in particular plasma lipoprotein particles.

In particular, the method may comprise:
a. Contacting the biological sample with an antibody specific for the flaviviral NS1 to form a first immunoreaction product;
b. Contacting said first immunoreaction product with an antibody specific for plasma lipoproteins to form a second immunoreaction product;
c. detecting the presence of the second immunoreaction product, and
d. quantitating the complex formed by NS1 and plasma lipoprotein particles.

The complex may be formed by the flaviviral non-structural protein 1 (NS1) and endogenous lipoprotein particles present in plasma selected among high-density lipoprotein particles (HDL), low-density lipoprotein particles (LDL), intermediate-density lipoprotein particles (IDL), very low-density lipoprotein particles (VLDL) and chylomicrons.

The complex may be formed by a flaviviral NS1 and LDL particle which bind together with a lower affinity than NS1 with HDL particle (equilibrium dissociation constant K_{D} of 1.4 nM). The complex may be formed particularly by a flaviviral NS1 and an apolipoprotein B (ApoB)-positive lipoprotein particle or by a flaviviral NS1 and an apolipoprotein E (ApoE)-positive lipoprotein particle.

According to a particular aspect of said method, the complex is formed by a flaviviral NS1 and HDL particle which bind together with a strong affinity (equilibrium dissociation constant K_{D} of 63.8 nM). According to a more particular aspect of said method, the complex is formed by a flaviviral NS1 and an apolipoprotein A1 (ApoA1)-positive lipoprotein particle.

*Flaviviral* and flavivirus according to the present invention refers to and encompasses in particular Dengue virus, West Nile virus, Japanese encephalitis virus, Zika virus and Yellow fever virus. According to an advantageous embodiment of the invention, the flavivirus is a dengue virus.

The term "lipoprotein particle" is used interchangeably with "lipoprotein" in the present description and encompasses high-density lipoproteins (HDL), low-density lipoproteins (LDL), intermediate-density lipoproteins (IDL), very low-density lipoproteins (VLDL) and chylomicrons. The terms "HDL", "LDL", "VLDL", "IDL" are used interchangeably with "HDL particle", "LDL particle", "VLDL particle", "IDL particle" respectively. "NS1-ApoA1 complex", "NS1-ApoE complex", "NS1-ApoB complex" refers to a complex of NS1 and ApoA1, ApoE, and ApoB positive apoliprotein particle, respectively. The used antibodies can be synthetic, monoclonal, or polyclonal and can be made by techniques well known in the art. Such antibodies specifically bind via the antigen-binding sites of the antibody (as opposed to non-specific binding). The monoclonal antibodies include antigen-binding fragments, and chimeric antibodies, e.g., humanized versions of murine monoclonal antibodies.

According to a particular aspect of said method, the antibody specific for the flaviviral NS1 is used as a capture antibody and the antibody specific for plasma lipoprotein particles is used as detection antibody.

According to a particular aspect of said method, the antibody specific for the flaviviral NS1 is coated on a solid support and the antibody specific for plasma lipoproteins is a detection or revelation antibody. This revelation antibody is optionally conjugated to a suitable label.

According to another particular aspect of said method, when the antibody specific for plasma lipoproteins is not conjugated to a label, a third antibody raised against this antibody and conjugated to a suitable label is used for detecting the presence of the second immunoreaction product. Said third antibody is a conventionally used antibody, such as for example an IgG directed against this antibody and produced in particular in goat, pig or donkey. Among the labels used, mention may be made of fluorescent label, biotin/streptavidin system, nonisotopic labels or enzymes, such as for example horseradish peroxidase or alkaline phosphatase.

The antibody specific for plasma lipoproteins may be an antibody specific for ApoE.

According to another particular aspect of said method, the antibody specific for plasma lipoproteins is an antibody specific for Apolipoprotein A1 (ApoA1).

When the plasma lipoprotein particles are Low Density Lipoproteins (LDL) particles, the antibody specific for plasma lipoproteins can be an antibody specific for apolipoprotein B.

The *in vitro* method for quantitating a complex formed by the flaviviral non-structural protein 1 (NS1) and endogenous lipoprotein particles, in particular plasma lipoprotein particles may comprise : (i) contacting firstly the biological sample with an antibody specific plasma lipoproteins and contacting secondly the biological sample with an antibody specific for flaviviral NS1; or (ii) contacting the biological sample concurrently with an antibody specific plasma lipoproteins and an antibody specific for flaviviral NS1.

In some embodiments of the invention, the diagnostic, prognostic or monitoring of a flaviviral infection or associated disease is performed using the *in vitro* method for quantitating a complex formed by the flaviviral non-structural protein 1 (NS1) and plasma high-density lipoprotein (HDL) particles as disclosed herein; preferably a complex formed by the flaviviral NS1 and apolipoprotein A1 (ApoA1)-positive lipoprotein particles as disclosed herein; preferably wherein the antibody specific for plasma lipoproteins is an antibody specific for Apolipoprotein A1 (ApoA1). In some preferred embodiments of the diagnostic, prognostic or monitoring method according to the invention, the quantity of the complex is determined by capture ELISA using the antibody specific for the flaviviral NS1 coated on a solid support and the antibody specific for plasma lipoproteins as detection antibody; and/or a third antibody raised against antibody specific for plasma lipoproteins and conjugated to a suitable label is used for detecting the presence of the second immunoreaction product.

An embodiment of the invention concerns an *in vitro* prognostic method of a severe form of a flaviviral infection in a subject infected with a flavivirus, comprising carrying out the *in vitro* method for quantitating a complex NS1-plasma HDL particles, as described above, in a biological sample obtained from a subject after infection, and preferably during primary or acute infection, and wherein the more the level of complex, the less the risk to develop severe form of flaviviral infection. In a preferred embodiment of said method, the complex is formed by flaviviral NS1 and ApoA1-positive lipoprotein particles.

Another embodiment of the invention concerns an *in vitro* method for monitoring a flaviviral disease in a subject infected by a flavivirus, comprising carrying out the method for quantitating a complex NS1-plasma HDL particles, as described above, on biological samples obtained from said subject at different times during the flaviviral disease.

Another embodiment of the invention concerns an *in vitro* method for diagnosing a flaviviral infection in a subject, comprising carrying out the method for quantitating a complex NS1-plasma HDL particles, as described above, on a biological sample obtained from said subject, wherein the presence complexes formed by flaviviral NS1 and plasma lipoprotein particles in said sample is indicative of a flaviviral infection.

According to a particular embodiment of the *in vitro* method for diagnosing a flaviviral infection, complexes formed by flaviviral NS1 and HDL particles are quantitated, preferably complexes formed by flaviviral NS1 and ApoA1-positive lipoprotein particles.

According to another particular embodiment of the *in vitro* method for diagnosing a flaviviral infection, the method for quantitating a complex NS1-plasma lipoprotein particles, as described above, is repeatedly carried out on a biological sample obtained from said subject to quantitate complexes of flaviviral NS1-HDL particles, preferably complexes of flaviviral NS1-ApoA1-positive lipoprotein particles and complexes of flaviviral NS1-ApoE-positive lipoprotein particles.

The invention also concerns the use of the presence or level of a complex formed by a flaviviral non-structural protein 1 (NS1) and plasma high-density lipoprotein particles, particularly NS1-ApoA1-positive lipoprotein particle complex, as a biomarker for the diagnostic, prognostic or monitoring of a flaviviral infection or associated disease according to the present disclosure, particularly a dengue virus infection or associated disease. In some aspects, NS1-HDL particle complex, particularly NS1-ApoA1-positive lipoprotein particle complex is used as a biomarker for the prognostic of the flaviviral infection or associated disease. In some other aspects, NS1-ApoA1, NSI-ApoE and NS1-ApoB positive lipoprotein particle complexes are used as biomarkers for the diagnostic of the flaviviral infection or associated disease. In some other aspects, NS1-ApoA1 and NSI-ApoE-positive lipoprotein particle complexes are used as biomarkers for the monitoring of the flaviviral infection or associated disease.

The invention also relates to a kit for the diagnostic, prognostic or monitoring of a flaviviral infection or associated disease in a biological sample obtained from a subject, comprising:
a. An antibody specific for the flaviviral NS1;
b. An antibody specific for plasma lipoproteins which is specific for HDL, preferably for ApoA1; and further comprising an antibody specific for ApoB and/or ApoE;
c. Means for detecting the production of an immunoreaction product between said two antibodies and the complex formed by the flaviviral NS1 and plasma lipoprotein particles.

The kit comprises several antibodies specific for plasma lipoproteins, each of these antibodies being specific for one population of plasma lipoproteins.

The antibody specific for ApoA1 is specific for HDL as ApoA1 protein is carried by HDL.

The antibody specific for ApoB protein is specific for LDL as ApoB protein is carried by LDL.

The antibody specific for the ApoE protein recognizes several populations of plasma lipoproteins.

The antibody specific for the flaviviral NS1, as the antibodies specific for plasma lipoproteins used in the kit according the invention is a polyclonal antibody, a monoclonal antibody, or antigen-binding portions thereof.

The antibodies specific for plasma lipoproteins may carry a detectable label.

Said means for detecting the production of an immunoreaction product may comprise a third antibody directed against one antibody specific for plasma lipoproteins and conjugated to a suitable label.

The kit may further comprise instructions for use of said kit.

The kit may further comprise at least one reference sample. This reference sample can be a sample of plasma from healthy donors, from patients for which lipid markers had returned back to normal at the time of discharge and had essentially recovered from the disease, or a sample of plasma from patients hospitalized without delay after the onset of fever (i.e. no more than 3 days of fever at admission), spiked with a known quantity of purified flaviviral NS1.

This reference sample allows to more reliably define the positive threshold value to be considered for the assay.

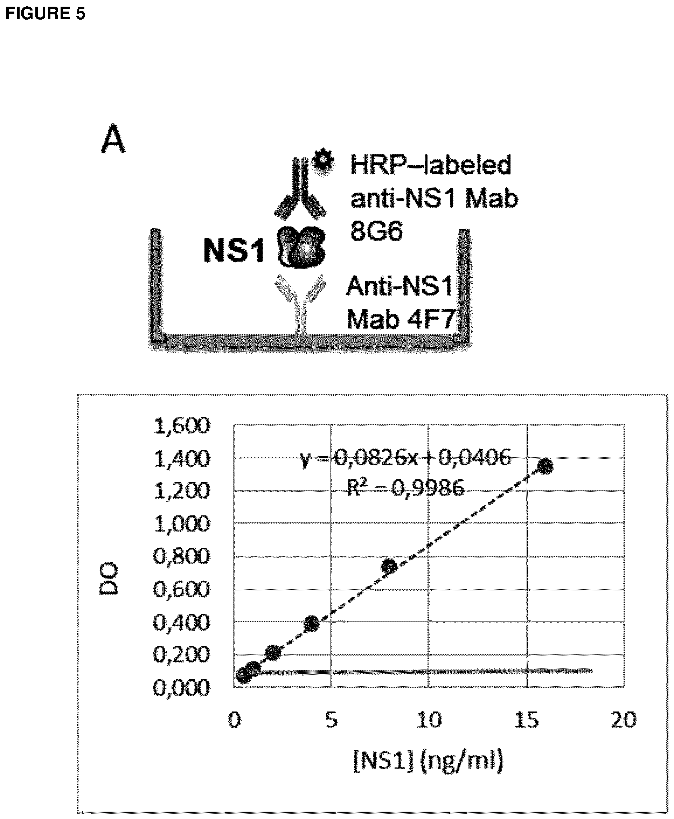

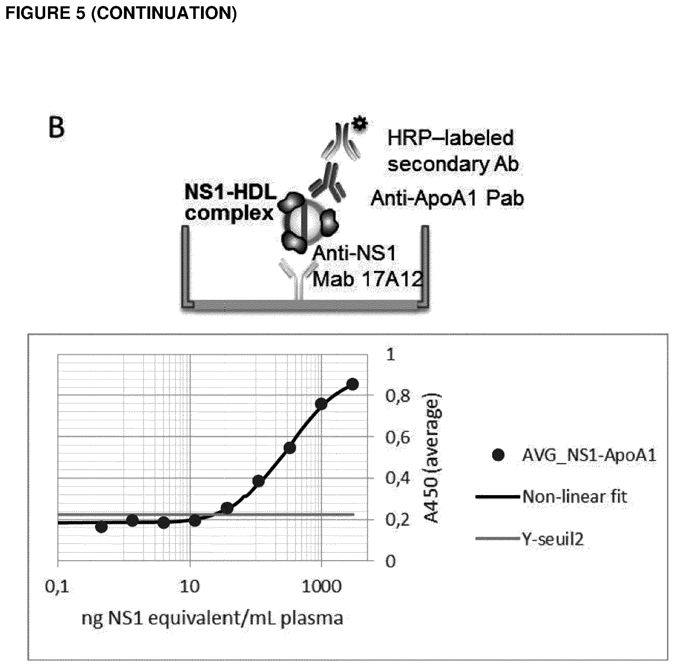

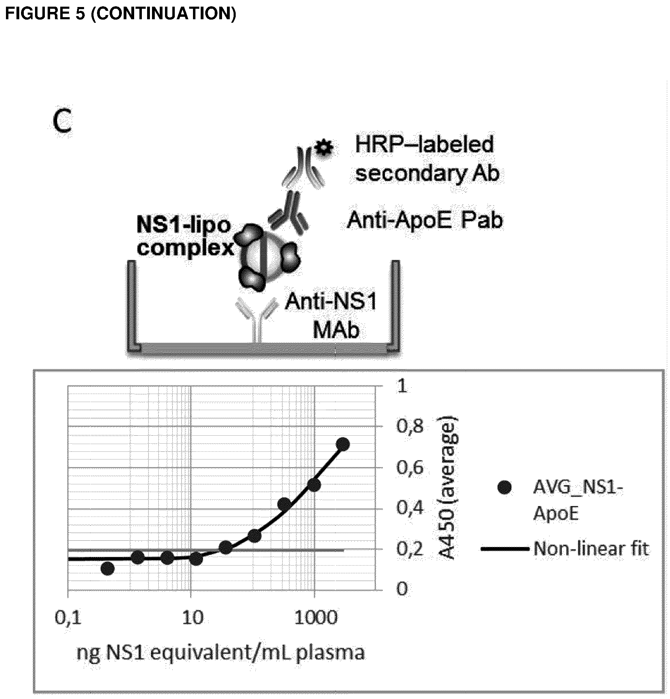

For NS1-ApoA1 and NS1-ApoB assays, calculation of the positive threshold value is based on the principle that the patients for which lipid markers had returned back to normal at the time of discharge (i.e. Tot-Chol>3 mmol/mL, LDL-Chol>1.5 mmol/mL, HDL-Chol>0.4 mmol/mL, Trig.<2.7 mmol/mL; n=9) had essentially recovered from the disease and turned negative for the NS1-ApoA1-positive complex (Fig. 5). The positive threshold was then considered as twice this average value. The strategy to estimate the positive threshold of NS1-ApoE assay differs due to the fact that the trend for the formation/accumulation of this complex is opposite to the NS1-ApoA1 complex with a global increase of the NS1-ApoE concentrations over time. The calculation of the mean value is based on a group of patients that was hospitalized without delay after the onset of fever (i.e. no more than 3 days of fever at admission; n=11).

The diagnostic method of a flavivirus infection or associated disease according to the invention may be used to determine if a subject is in need of an appropriate treatment. The treatment may be any suitable therapy for treating flaviviral infections that is well-known in the art such as antiviral therapy, immunotherapy and combination thereof; in particular using antibodies blocking the formation of complex between NS1 and endogenous lipoproteins or using exogenous lipoprotein particles.

The detection of the level of NS1-HDL complex according to the method of the invention, wherein a higher level of NS1-HDL complex is indicative of a lower risk to develop severe form of flaviviral infection, allows advantageously to adapt the treatment of the individual depending upon the severity of the disease. The complex is preferably NS1-ApoA1-positive lipoprotein particle complex. The detection is preferably performed during primary or acute infection, as disclosed above.

The monitoring of the flaviviral disease in a subject infected by a flavivirus by carrying out the method for quantitating a complex NS1-plasma lipoprotein particles, as described above, on biological samples obtained from said subject at different times during the flaviviral disease is useful in the treatment of the infected subject.

In a particular embodiment of the invention, the flaviviral NS1 is the NS1 from Dengue virus and/or the flaviviral infection is a Dengue virus infection.

In a particular embodiment of the invention, the biological sample is blood, plasma or serum.

In a particular aspect of the invention, the detection of the NS1-high-density lipoprotein complex is an early detection during the clinical phase of the flaviviral infection, in particular during primary or acute flaviviral infection.

In a particular embodiment of the invention, the subject is a human being.

The features described here-above and other features of the invention will be apparent when reading the examples and the figures, which illustrate the experiments conducted by the inventors, in complement to the features and definitions given in the present description. The examples arehowever not limitative with respect to the described invention.

### LEGEND OF THE FIGURES

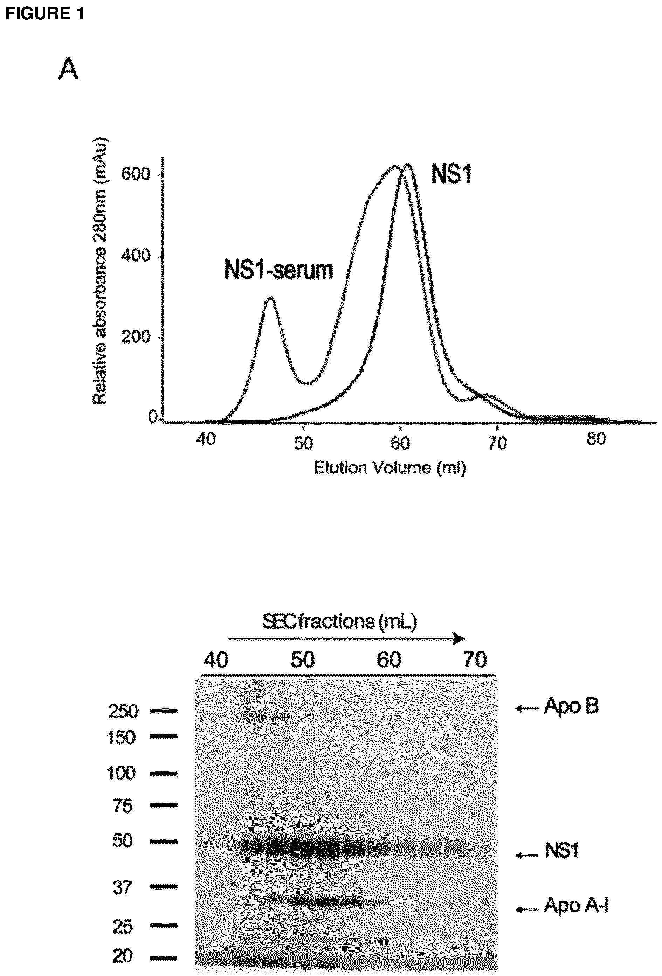

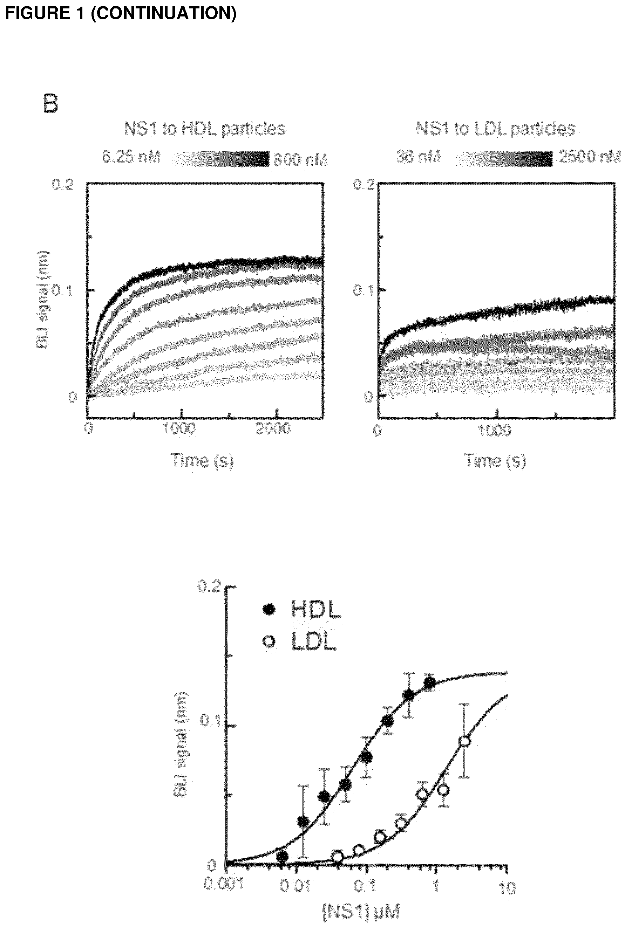

**Figure 1****. The dengue virus nonstructural NS1 binds to human HDL and LDL lipoprotein particles. (A)** Size exclusion chromatography (SEC) profile of NS1 pull-down experiments showing a clear shift to lower elution volumes after incubation in human serum (black line) compared to the NS1 protein alone (blue line). NS1 protein partners were identified in SDS-PAGE and N-terminal sequencing as the Apolipoprotein B-48, scaffold of the low density lipoproteins (LDL) in the first SEC elution peak, and the ApoA1 protein, scaffold of the high-density lipoproteins (HDL) in the second elution peak. (B) Biolayer interferometry signal for the titration of NS1 binding to HDL (left panel) and LDL (central panel) particles, respectively. Steady-state single molecule model of association fitted for the binding signal of NS1 to HDL (black dots) and LDL (white dots) is shown on the right hand side panel.

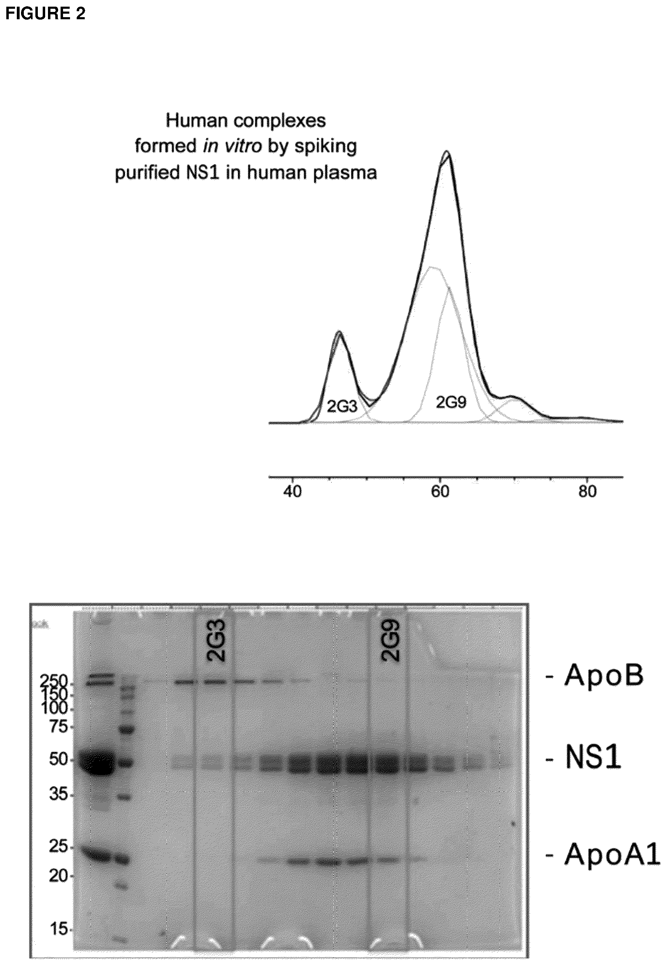

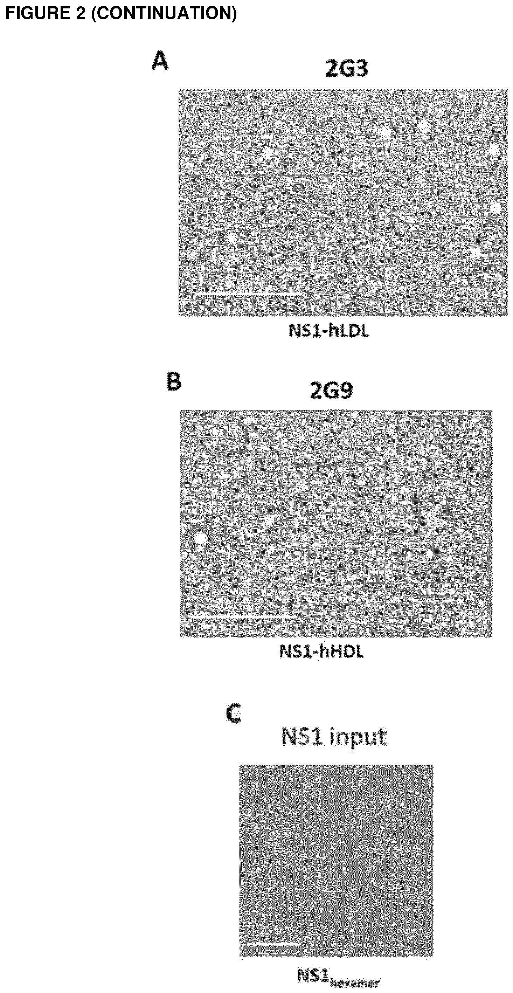

**Figure 2****. Visualization of the NS1-HDL and NS1-LDL complexes by electron microscopy.** The most representative fractions of the NS1-LDL (A), the NS1-HDL (B) and the purified NS1 input protein (C) were negatively stained with 2% uranyl formate and analyzed with a Tecnai G2 Bio-Twin electron microscope. Images were acquired with an Eagle camera and recorded in low-dose mode on a Falcon II direct electron detector.

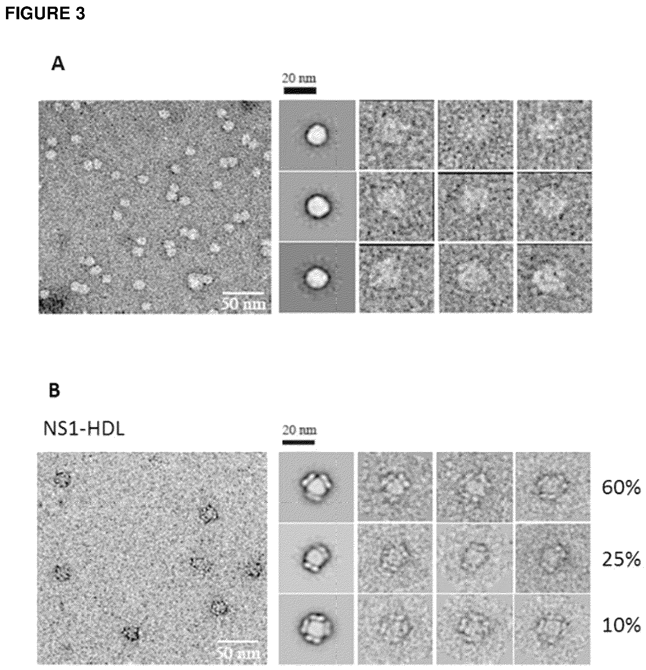

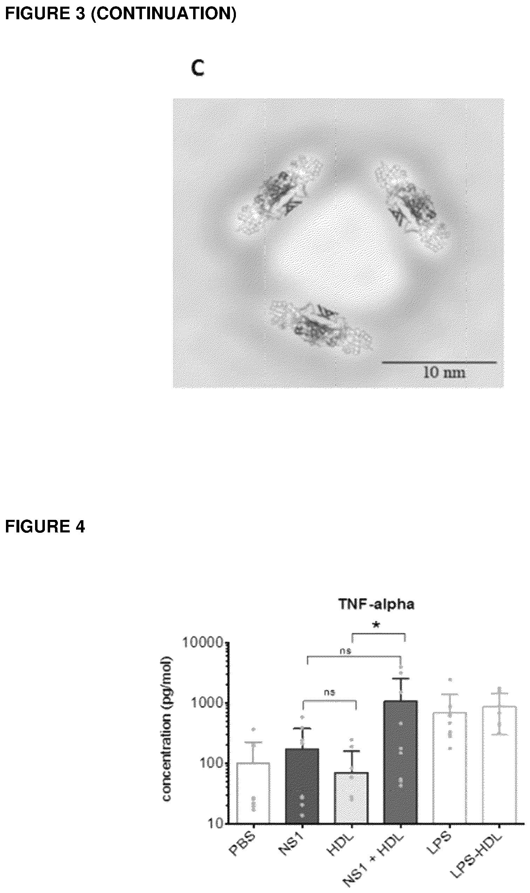

**Figure 3****. Image processing of the electron micrographs reveals the presence of NS1 dimers at the surface of HDL particles. (A, B)** Electron microscopy observations from left to right: a representative e-m image is shown, with the three most representative classes of purified HDL particles **(A)** and NS1-HDL complexes **(B). (C)** Fitting of the NS1 3D structure of the dimeric form into the most abundant class of NS1-HDL complexes.

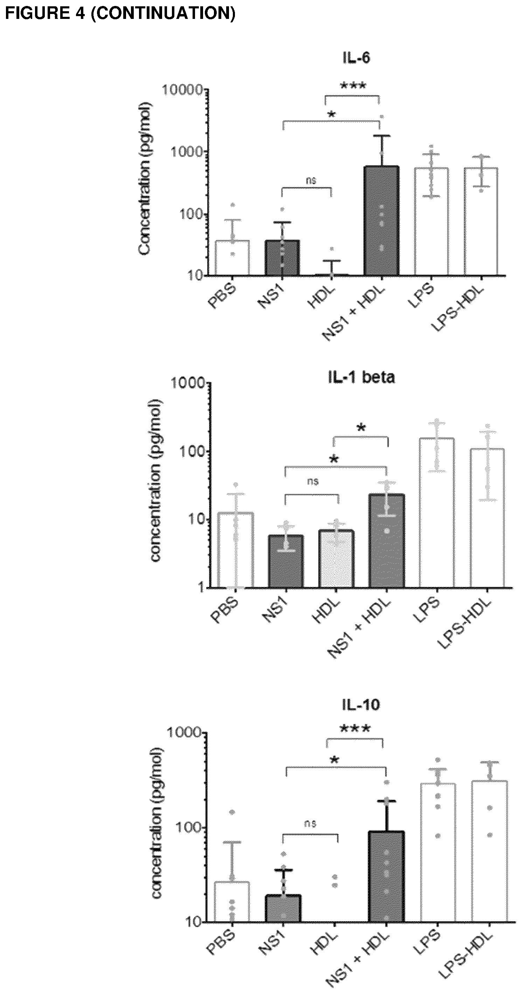

**Figure 4****. The NS1-HDL complex triggers the production of inflammatory cytokines in human primary macrophages.** Macrophages recovered from 4 different donors and incubated for 24h with the different effectors, as specified. LPS stimulation was used as a positive control. At the end of the incubation period, cell culture supernatants were recovered and concentrations of TNFα, IL-1β, IL-6 and IL-10 were quantified using Luminex assays. Data represent the mean values +/- SEM. Mean cytokine levels were compared using a 2-Way analysis of variance (Anova).
**Figure 5****. Standard ELISA for quantification of (A) the NS1 antigen, (B) the NS1-ApoA1/HDL complexes or (C) NS1-ApoE positive lipoprotein particles.** Schematic representations of the principle of the three different ELISA are given on the left hand side of the Figure. **(A)** Calibration curve of a dengue NS1 reconstituted *in vitro.* Purified DENV-NS1 was incubated at a known concentration in normal plasma obtained from a human donor for 1h30 at 37°C. DENV-NS1 was captured using an immobilized anti-NS1 monoclonal antibody (17A12) and bound NS1-mAb17A12 complexes further detected by peroxidase-labeled anti-dengue NS1 MAb (8G6) **(B)** Calibration curve of a dengue NS1-HDL complex reconstituted *in vitro* as for the DENV-NS1. The NS1-HDL complex was captured using an immobilized anti-NS1 monoclonal antibody (17A12) and bound complexes further detected with a commercial anti-ApoA1 polyclonal antibody followed by a species-specific peroxidase-labeled secondary antibody. (C) An anti-ApoE polyclonal antibody was alternatively used as a secondary antibody for the detection of NS1 complexes formed with ApoE. The concentration values reported on the x-axis are given as an NS1 equivalent concentration, an estimation that is based on our observation that all NS1 molecules bind to HDL present in large excess in this experimental setting. All curves are representative of at least 3 sets of experiments.

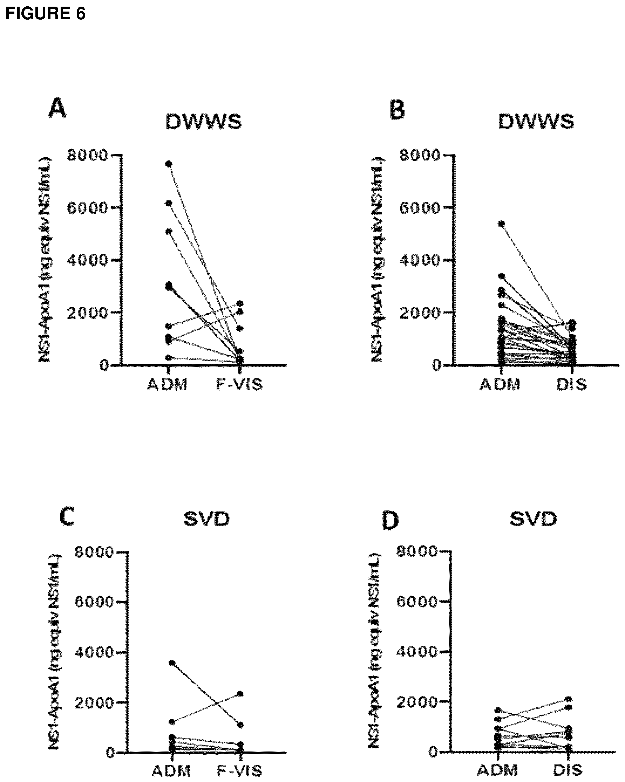

**Figure 6****: Detection of the NS1-ApoA1 complex in the plasma of hospitalized dengue virus-infected patients.** Apolipoprotein A1 is the scaffold protein of HDL. Concentrations of NS1-ApoA1 complexes are representative of the abundance of virolipoprotein NS1-HDL particles in plasma samples. NS1-ApoA1 complexes were quantified by ELISA as in Figure 5 in two groups of hospitalized patients that were classified as showing dengue with warning signs (DWWS, n=36; **panels A, B)** or severe dengue (SVD, n=19; **panels C, D).** The two groups were tested following admission at hospital (ADM) or during the follow up visit (F-VIS) **(panels A and C)** or on admission and discharge (DIS) for patients that recovered during their stay at hospital **(panels B and D).** On average, the two blood samples were recovered 3 to 4 days apart from each patient. The dotted line in each graph indicates the positive threshold.

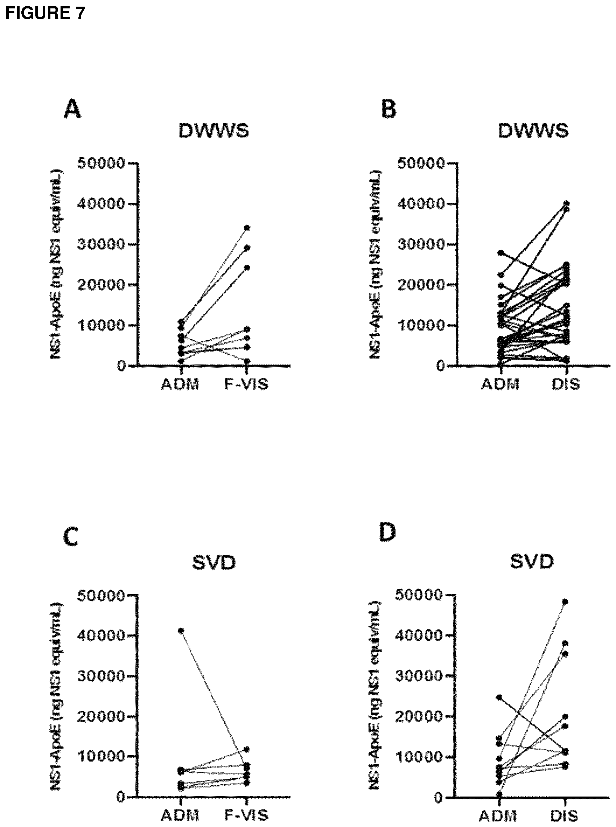

**Figure 7****: Detection of NS1-ApoE complexes in the plasma of hospitalized dengue virus-infected patients.** The samples described in Figure 6 were tested using an ApoE-specific ELISA. The assay described in Figures 5 and 6 was slightly modified by replacing the secondary anti-ApoA1 polyclonal antibody with a commercial anti-ApoE polyclonal antibody. The description of the four **panels A, B, C and D** is identical to Figure 6.

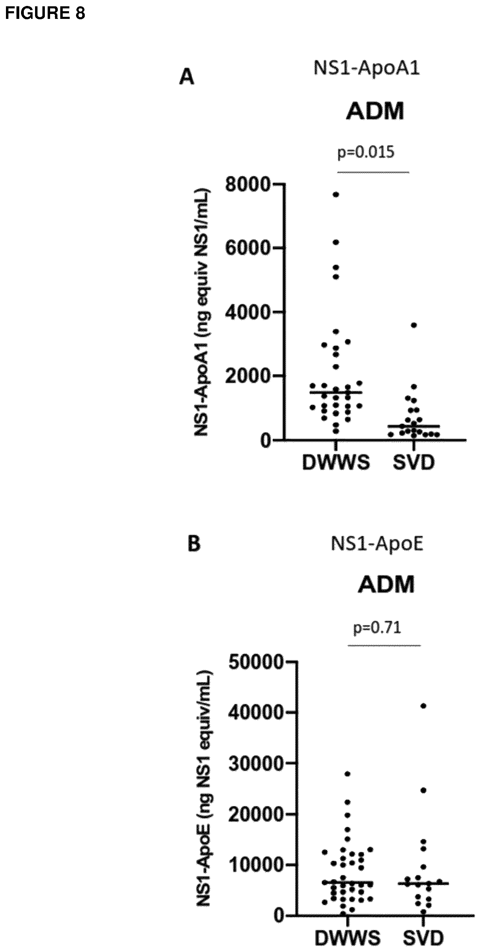

**Figure 8****. Correlation tests between the concentrations of NS1-ApoA1 and NS1-ApoE and the severity of the disease in dengue virus-infected patients showing either DWWS** or **SVD.** Concentrations of the NS1-ApoA1 **(panel A) or** NS1-ApoE **(panel B)** complexes are plotted separately for the two clinical groups of patients that developed either DWWS or SVD upon DENV infection. The mean values observed for the different parameters are shown by plain bars and compared using a 2-Way analysis of variance (Anova).

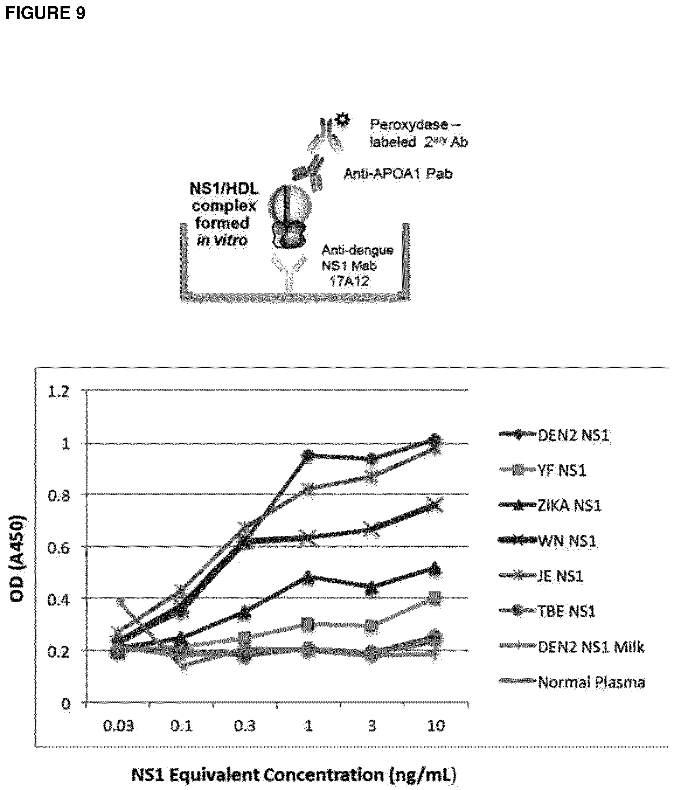

**Figure 9****. Capacity of NS1 protein to bind to HDL is shared among flaviviruses. ELISA detection of NS1-ApoA1 complex with NS1 from different flaviviruses. Purified NS1** from different flaviviruses (yellow fever, YF; ZIKA; West Nile, WN; Japanese encephalitis, JE; tick-borne encephalitis, TBE) were spiked for 1h30 at 37°C in normal plasma and NS1-HDL complexes were further detected by capture-ELISA. The standard curve was obtained using a dengue NS1-HDL complex reconstituted *in vitro.* The different flavivirus NS1-HDL complex were captured using an immobilized anti-dengue NS1 monoclonal antibody (17A12) as depicted on the Figure 5 and bound complexes further detected with a commercial anti-ApoA1 polyclonal antibody followed by a species-specific peroxidase-labeled secondary antibody. The concentration values reported on the x-axis are given as an NS1 equivalent concentration, an estimation that is based on our observation that all NS1 molecules bind to HDL present in large excess in this experimental setting.

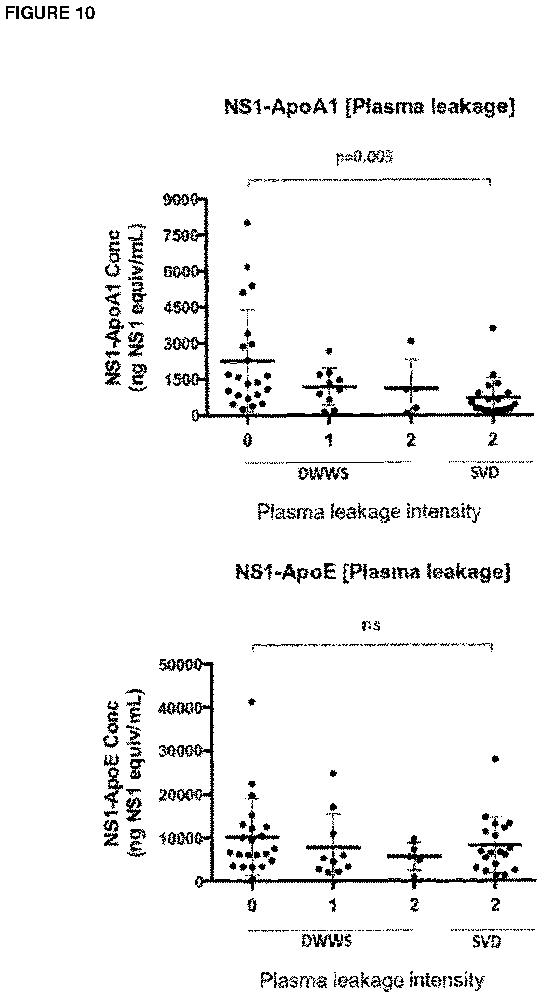

**Figure 10****: Correlation tests between the concentrations of NS1-ApoA1 and NS1-ApoE and plasma leakage intensity in dengue virus-infected patients on admission at hospital.**
   Concentrations of the NS1-ApoA1 (A) or NS1-ApoE (B) complexes are plotted against the intensity of pleural effusion as observed by ultrasonography and graded as 0 (no pleural fluid detected), 1 (moderate effusion) and 2 (abundant effusion) for patients with dengue warning signs (DWWS). All patients with severe dengue (SVD) were grade 2. Statistical differences could be observed between the DWWS-grade 0 and the SVD groups of patients for the NS1-ApoA1 complex (p=0.005) and not the NS1-ApoE complex (p=0.42; ns, not significant).

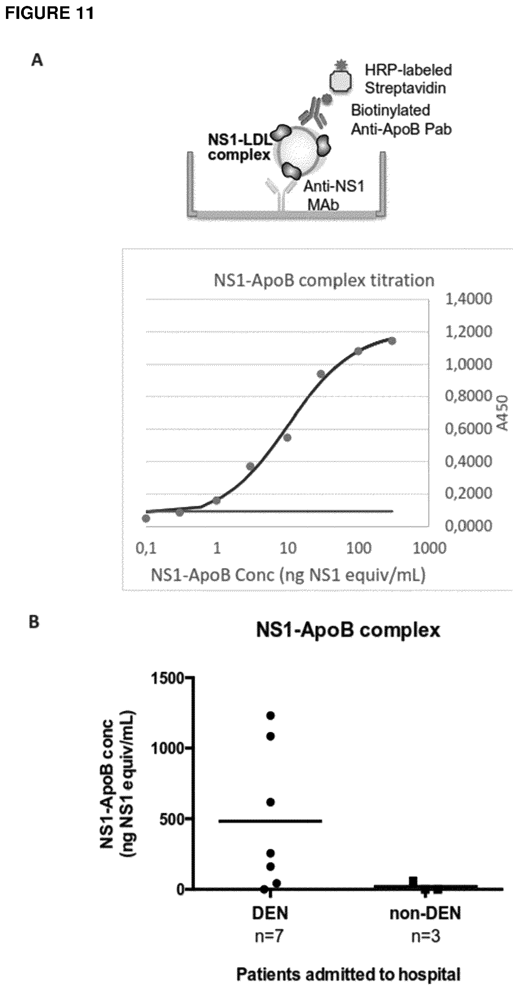

**Figure 11****: Detection of the NS1-ApoB complex in the plasma of hospitalized dengue virus-infected patients.**
   Apolipoprotein B is the scaffold protein of LDL. NS1-ApoB complexes were quantified by ELISA (A) (Material & Methods) in dengue virus-infected patients (n=7) or in non-dengue hospitalized patients (n=3). The two groups were tested for the presence of NS1-ApoB complexes on admission at hospital (ADM).

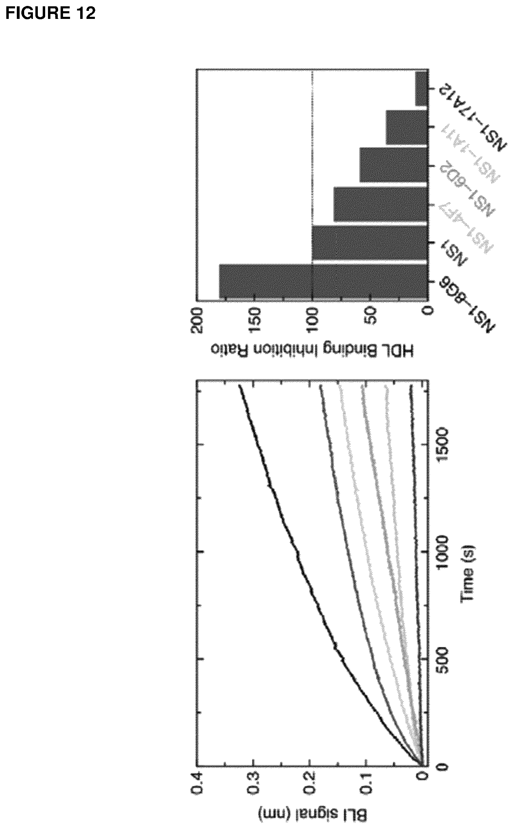

**Figure 12****: Association of the NS1-HDL lipoprotein complex can be blocked by NS1-specific MAbs.**
   Several anti-dengue NS1 MAbs were incubated with purified NS1 and the resulting immune complexes exposed to immobilized HDL. The residual binding capacity of NS1 was measured by BLI as in Fig. 1B. Purified NS1 alone was used as a positive control. Of note, none of the MAbs tested in the absence of NS1 bound to the HDL particles.

### EXAMPLES

### Material & Methods

### DENV or flavivirus NS1 spiking in human plasma

DENV2 recombinant NS1 protein (400µg) was incubated for 1h at 37°c in 1mL of serum or plasma obtained from healthy donors (provided by the Institut Pasteur IcareB biologicals facility). All human samples comply with ethical regulations.

### NS1 serum pull-down

The mix was then purified through a Strep-tactin column (Iba), washed twice with PBS MgCa (Gibco) followed by 14 column volumes of PBS 0.3 M NaCl and another 5 column volumes of PBS MgCa. Elution was performed using 2.5 mM D-desthobiotine (Iba) in PBS Mg/Ca.

### Size exclusion chromatography (SEC)

Gel filtrations of recombinant NS1, HDLs or NS1-HDL solution were done on a Superdex 200 10/300 column equilibrated in PBS MgCa++ at 0.4ml/min with recovery of 0.5ml fractions. The elution profiles were compared with standards from Bio-Rad.

### SDS-PAGE of NS1 and NS1-HDL SEC fractions

All the protein samples were denatured in 5x Laemmli Sample Buffer (Bio-Rad) containing β-mercaptoethanol and boiled for 5 min at 95 °C. They were separated by discontinuous sodium dodecyl sulfate (SDS) 10% polyacrylamide gel electrophoresis (SDS-PAGE precast gels, Bio-Rad). The SDS-PAGE gels were stained in Coomassie Blue solution (Bio-Rad).

### Biolayer Interferometry

The inventors titrated the DENV2 NS1 binding to HDLs or LDLs with the Octet Red (ForteBio) Biolayer Interferometry assay (BLI). Experiments were performed at 25 °C, at a shake speed of 1000 rpm in a 96-well plate.

HDLs had a uniform diameter of 9.6 nm and were commercially available (Merck Millipore). The Streptavidin A (SA) "dip and read" biosensors (ForteBio) were activated and loaded with the biotinylated anti-ApoA1 or anti Apo-B antibodies before loading the respective lipoproteins and subsequently enter the NS1 protein solutions. BLI signal for NS1 binding to the lipoproteins was monitored over time and controls for NS1 binding to the free tip and free antibodies were measured, as well as background noise from buffer interactions with the lipoproteins or the antibodies. The controls were subtracted from the corresponding signals for each NS1 concentration. Binding signals were analysed using the Scrubber sotfware for data extraction and normalisation, BlAevaluation (BIACORE), for data control subtraction and Profit (Quantumsoft), for data fitting.

### Capture ELISA of NS1-ApoA1 or NS1-ApoE lipoprotein complex

Briefly, microtitration plates were coated overnight with immuno-affinity purified mouse anti-NS1 polyclonal antibodies or with Dengue NS1-specific monoclonal antibody 17A12 (deposited under the terms of the Budapest Treaty at the Collection Nationale de Culture de Microorganismes (CNCM) on 4 March 2004, under the number I-3186). ). Wells were saturated and washed before serial dilutions of human sera spiked with purified dengue virus type 1 NS1 or dengue virus-infected human sera were added to wells for 2 h at room temperature. Wells were washed again and incubated for 1 h at 37°C with anti-ApoA1 or anti-ApoE goat polyclonal antibodies followed by a peroxidase-conjugated secondary antibody revealed with a 3,3", 5,5"-tetramethylbenzidine solution. Negative controls were measured when the reaction was carried out in the absence of antigen. Absorbance values were corrected by using the mean value of the negative controls. Healthy donors were recruited by the IcareB Platform of Institut Pasteur.

### Detection of NS1-ApoB lipoprotein complexes by capture ELISA

The protocole used is depicted in Figure 11 (A). Microtitration plates were coated overnight with purified mouse anti-NS1 polyclonal antibodies or anti-NS1 monoclonal antibodies. Wells were saturated and washed before serial dilutions of the dengue virus-infected human sera or control patients infected with other pathogens were added to wells for 2 h at room temperature. Wells were washed again and incubated for 1 h at 37°C with anti-ApoB biotinylated goat polyclonal antibodies followed by peroxidase-conjugated Streptavidin revealed with a 3,3", 5,5"-tetramethylbenzidine solution. Quantification of the NS1-ApoB complexes reported in Figure 11 (B) was based on a standard curve obtained with normal human plasma spiked with known concentrations of purified recombinant NS1 antigen (A).

### Dengue-virus infected patient sera specimen

Human sera were collected from dengue virus type 1-infected patients during an epidemic in Cambodia in 2011-2012. Dengue virus diagnosis was confirmed by RT-PCR on blood samples recovered on the first day of hospitalization. Each patient was also tested by MAC-ELISA and hemagglutination inhibition (HI) tests on paired sera (acute and convalescent phases). Various clinical and biochemical informations were available for patients included in the study, which allowed their classification in the different groups of disease severity according to the 2009 WHO classification system. No classical dengue fever was observed in the hospitalized patients classified with the 2009 classification, about 2/3 of the patients had dengue with warning signs (DWWS) and 1/3 developed severe dengue (SVD).

### Electron microscopy imaging

Purified HDLs or NS1-HDL complex was spotted on glow discharged carbon grids (CF300, EMS, USA), negatively stained with 2% uranyl formate (UFA) pH 7.4, analyzed with a Tecnai G2 Bio-Twin electron microscope (FEI, USA) and imaged with an Eagle camera (FEI, USA). Image frames were recorded in low-dose mode on a Falcon II direct electron detector (FEI, USA).

### Image analysis

HDL and NS1-HDL negative staining images were CTF-corrected (phase flip) and sorted using the XMIP software (Scheres et al. 2005). Corrected images were imported in Relion (Scheres 2012). The recommended strategy for particle picking was applied as follow: a manual selection of particles compatible with the HDL or NS1-HDL size was performed on a small number (about fifteen) of images. A 2D classification (40 classes) was performed and five representative well defined classes were selected as template for the automatic picking, leading to about 30 000 particles. A 2D classification (200 classes) was then performed. Classes obviously corresponding to artefacts were suppressed and a new run of 2D classification (200 classes) was performed.

### Primary monocyte culture and differentiation into macrophages

PBMCs were isolated from whole blood using a Ficol gradient centrifugation ( Eurobio). CD14+ cells are purified by magnetic bead separation of PBMCs using CD14+ human positive selection kit (StemCell) and plated 1M/ml on teflon plates (Sarstedt,) with 7ml per plate in the following medium : RPMI-1640 (Gibco), 2mM L-glutamine (LifeTechnologies), 1% penicillin-streptomycin of concentration: 10, 000 units penicillin and 10 mg streptomycin/ml (LifeTechnologies), 10mM Na Pyruvate (LifeTechnologies), 10mM HEPES (LifeTechnologies), 1% MEM vitamins from stock (LifeTechnologies), 1% NEAA (LifeTechnologies), 50 uM beta-mercaptoethanol (LifeTechnologies), 15% human serum. Monocytes were cultured in differentiating media for 6-8 days after which the macrophages were scraped off teflon plates and counted. After spinning, they were resuspended at 1M/ml in the same media but with 10% FBS instead of human serum.

### Macrophages immune activation assay

Macrophages were plated at 0.5.10^6 cells/mL in P24 plates (Corning). After 2 hours sedimentation and adhesion of the cells, macrophages were incubated with simple PBS, HDL, NS1 or a NS1-HDL mix for 24h before collection of the supernatants. Luminex 5-plex assay was performed on all supernatants according to the manufacturer's recommendations (R&D systems human 5-plex kit). Standards were run with each plate at every assay to titrate the level of cytokines present.

### Statistical analysis

Significant differences between groups were determined by One-way ANOVA analysis performed with GraphPad Prism software.

### Antibodies

Anti-ApoA1 goat polyclonal antibodies are provided by Novus Biologicals (Product #NB400-147).

Anti-ApoE goat polyclonal antibodies are provided by Calbiochem (Product #178479).

Peroxidase-conjugated secondary antibody is provided by Southern Biotech (Product #6425-05).

Anti-ApoB biotinylated goat polyclonal antibodies are provided by ABCAM (Product #ab20898).

Peroxidase-conjugated streptavidin is provided by Interchim (Product #396888).

Biotinylated antibodies against ApoA1 or ApoB are provided by ABCAM (respectively Product #ab27630 and Product #ab20898).

Mouse anti-NS1 polyclonal antibodies are obtained as described by Alcon-LePoder *et al.* 2006.

Murine hybridoma cell culture secreting the following dengue NS1-specific monoclonal antibodies were deposited at the Collection Nationale de Cultures de Microorganismes (Paris - France) under the following accession numbers:

| | |
|---|---|
| mAb 17A12 | I-3186 on 4 March 2004 |
| mAb 8G6 | I-5291 on 13 March 2018 |
| mAb 1A11 | I-5290 on 13 March 2018 |
| mAb4F7 | I-3185 on 4 March 2004 |

### Example 1: DENV NS1 binds preferentially to human high-density lipoproteins - Identification of novel NS1 protein ligands

The inventors carried out a pull-down assay using a purified preparation of a tagged-version of DENV2 NS1 in serum obtained from healthy human donors and analyzed the resulting products by size exclusion chromatography (SEC) **(****Fig. 1A****).** Compared to the NS1 protein alone, the pull-down SEC profile showed an additional peak and a large shoulder at lower elution volumes **(****Fig. 1A****).** The protein contents were analyzed by SDS-PAGE and the identity of the predominant protein bands was determined by mass spectrometry. The band migrating with a molecular weight (MW) of 29 kDa corresponded to Apolipoprotein A1 (ApoA1) and the one of high MW to Apolipoprotein B-48 (over 250 kDa). These two major species represent the main scaffold proteins of high-density lipoproteins (HDL) and low density lipoproteins (LDL), respectively **(****Fig. 1A****).**

### Example 2: DENV NS1 binds preferentially to human high-density lipoproteins - Binding affinity measurements by biolayer interferometry

The inventors used biolayer interferometry (BLI) to assess the relative affinity of NS1 for HDL and LDL particles **(****Fig. 1B****).** Purified HDL and LDL particles were immobilized on biosensors coated with streptavidin and further loaded with biotinylated antibodies against ApoA1 or ApoB, respectively. NS1 titration binding experiments against the HDL or LDL loaded biosensors presented a *bona fide* amplitude of interferometry signal with respect to the background **(****Fig. 1B****).** On the right hand side of **Fig. 1B** are reported the equilibrium values for NS1 titration to LDL and HDL with the deviations observed in at least three experiments. The inventors interpreted the titration values by fitting the data to a steady state single site binding model in order to compare the equilibrium dissociation constants of NS1 for HDL and LDL, of K_{D} of 63.8 nM and 1.4 µM, respectively. This difference suggested that NS1 preferentially binds HDL. Remarkably, the NS1-HDL complex remains stable over a period of 6 days (data not shown), suggesting that once formed the protein remains tightly associated to the lipoprotein particles.

### Example 3: DENV NS1 binds preferentially to human high-density lipoproteins - Visualization of the NS1-HDL complex by electron microscopy

The inventors further analyzed the complexes by negative stain electron microscopy **(****Fig. 2****,** **Fig. 3****).** As previously shown by others, human HDL and LDL particles appear as smooth spheres of about 10 nm and 20 nm in diameter, respectively (Zhang et al. 2015). The NS1-HDL and NS1-LDL complexes have sizes that are in concordance with these values **(****Fig. 2****).** However, the NS1-HDL complex contrasted with the smooth appearance of HDL alone and rather presented a granular surface with prominent rod-shaped structures on their surface **(****Fig. 3****).** These blobs fit well with the dimensions of the NS1 dimers. The dimeric form of NS1 is known to expose a large hydrophobic patch on one of its face. NS1 dimers could therefore directly insert the lipidic phase of HDL, a feature that confers the complex a particularly high stability.

### Example 4: DENV NS1 binds preferentially to human high-density lipoproteins - Characterization of a biological activity associated with the NS1-HDL complex

HDLs are potent modulators of inflammation, and in physiological conditions these lipoproteins are essentially anti-inflammatory regulators. On the contrary, NS1 is known to trigger the secretion of pro-inflammatory cytokines in macrophages (Modhiran et al. 2015). In order to establish whether NS1 requires binding to HDL to become functional, the inventors compared the activation state of macrophages treated with the different effectors. The inventors used human primary macrophages differentiated from monocytes isolated from various donors and stimulated the cells with NS1 alone (10 µg/mL), HDL alone (2.5 µg/mL), NS1-HDL (mix of 10 and 2.5 µg/mL, respectively) and LPS as a positive control. Supernatants were recovered after a 24 h incubation period and the respective levels of IL-1 beta, TNF-alpha IL-6 and IL-10 measured using a Luminex assay **(****Fig. 4****).**

For NS1 and HDL alone, the inventors observed no differences in cytokine levels compared to the negative control **(****Fig. 4****),** whereas LPS consistently induced high cytokine levels (data not shown). These observations ruled out any cytotoxic effects from background contaminants in the NS1 and HDL purified samples and indicated that the NS1 itself was not capable of inducing pro-inflammatory activation of human macrophages. In contrast, the NS1-HDL complex increased substantial levels of cytokine secretion in the different donor cells when compared to HDL or to NS1 alone **(****Fig. 4****).** This demonstrated that the NS1-HDL complex is the bioactive form of the protein and a potent modulator of inflammation.

### Example 5: Quantification of viral or host factors in the plasma of DENV-infected patients - Quantification of NS1 and NS1-lipoprotein complexes in plasma

Next the inventors assessed the presence of NS1-HDL complexes in DENV-infected patients in comparison to the secreted form of NS1. For this purpose, they used their classical ELISA for the NS1 detection (Alcon-LePoder *et al.* 2006) and they developed two ELISA to specifically detect NS1 complexes in human plasma samples. In the first assay, they used an anti-NS1 monoclonal antibody for the capture of NS1-HDL complexes and an anti-ApoA1 polyclonal antibody for the detection of bound material **(****Fig. 5****).** An anti-ApoE polyclonal antibody was alternatively used as a secondary antibody for the detection of NS1 complexes possibly formed with other types of lipoprotein particles as well (IDL, VLDL, chylomicrons and in certain instances HDL and LDL as well, see the 2019 review from D. Marais in *Pathology).* To set up the standard curves in an analytical format, purified NS1 was incubated in plasma from healthy donors obtained at ICAReB (Institut Pasteur) and concentration values of NS1-ApoA1, NS1-ApoB or NS1-ApoE complexes were calculated on the basis of an equivalent concentration of NS1 **(****Fig. 5****;** **Fig. 11****).** The detection limit of the NS1, NS1-ApoA1 or NS1-ApoE assays was set as twice the mean value of signals obtained with normal human plasma, which corresponded to 0.5, 0.3 and 15 ng of an equivalent NS1 concentration per milliliter, respectively **(****Fig. 5****;** **Fig. 11****).**

Patients presenting with acute dengue-like illness - between June and October of 2011 and 2012 - were enrolled at Kampong Cham Referral Hospital. Inclusion criteria, following the WHO 1997 classification scheme, were children between 2 and 15 years old who had fever or history of fever at presentation and onset of at least two of the following symptoms within the previous 72 hours: headache, retro-orbital pain, muscle pain, joint pain, rash, or any bleeding signs. The inventors performed a prospective, monocentric, cross-sectional study of hospitalized children with severe and non-severe dengue. The first visit was conducted at hospital admission (Visit 1, V1). The day of onset of symptoms was defined as day 0 of the illness. Visit 2 (V2) was conducted at the defervescence phase, which is characterized as the first day with temperature ≤ 38 °C. Finally, Visit 3 (V3) was performed and considered as the discharge visit for patients who recovered entirely, or as a follow-up visit for patients in the critical phase. A clinical and biological follow-up including abdominal/chest ultrasound recording was conducted at each visit. DENV infection of hospitalized patients was confirmed by NS1 antigen detection using our NS1-capture ELISA (Alcon-LePoder *et al.* 2006) and/or qRT-PCR and/or virus isolation on *Aedes albopictus* C6/36 cells on the plasma sample obtained at Visit 1 (Andries *et al.* 2015). Finally, the severity of the disease among confirmed dengue patients was assessed according to the WHO 1997 and 2009 criteria using clinical and biological data recorded at admission (ADM), follow up visit (F-VIS) or discharge (DIS). A total of 56 patients were classified retrospectively in 3 groups with increasing grades of disease severity according to the 1997 classification, i.e. dengue fever (DF, n=20), dengue hemorrhagic fever (DHF, n=29) and dengue shock syndrome (DSS, n=7). Quite notably, patients segregated only into 2 groups in the 2009 classification: dengue with warning signs (DWWS, n=19) and severe dengue (SVD, n=37), while no patient had classical dengue fever (DF).

The inventors measured the concentration of the NS1-ApoA1 and NS1-ApoE complexes in the Cambodian cohort one day after admission and discharge from hospital, which represented on average a time lag of 4.3 days between the two blood samplings **(****Fig. 6****,** **Fig. 7****).** The inventors separated patients that had a follow-up visit (F_VIS) as the last medical check-up and patients that were tested on admission and discharge, as well as discriminated cases of DWWS from SVD. The vast majority (around 90%) of the DWWS patients showed a sharp decrease in the amount of NS1-ApoA1 in blood between the first and the last samplings while half of the SVD patients showed a slight increase between the two time points **(****Fig. 6****).** On reverse, concentrations of NS1-ApoE increased significantly in the majority of samples over time **(****Fig. 7****),** suggesting a dynamic process taking place during the acute phase of the disease.

The inventors observed over 82% positive samples for the NS1-ApoA1 complex at admission and a remaining 36% positive samples on the third visit (acute/discharge) **(Table 1).** A reverse trend was observed for the NS1-ApoE complex, with a clear increase of their concentration over time and a rise from 25% to 50% between admission and last visit **(Table 1).** The inventors found that about 70% of the patients were positive in NS1 antigen over the window of 2 to 6 days after the onset of fever, corroborating numerous reports including ours, while only 42% of the same individuals were still positive in NS1 at the time of the last medical check-up or discharge **(Table 1).** Accordingly, concentrations of NS1 were found to decrease over time **(Table 1).** Overall, the NS1-ApoA1 complex appears to represent a better diagnostic candidate marker than the NS1 protein itself.

The inventors further demonstrated the presence of the NS1-ApoB complex in 5 out of 7 patients, while 3 control non-dengue patients were found negative **(****Fig. 11****).** The concentrations ranged from 162 ng equivalent NS1/mL to 1230 ng equivalent NS1/mL. Extended studies are needed to determine the added value that this parameter could offer in terms of dengue diagnosis and prognosis. **Table 1:** Estimates of the percentages of positive plasma samples according to the different virological parameters tested as diagnostic markers in the hospitalized dengue virus-infected patients. Values are reported for patients that were admitted at hospital (ADMISSION), or at the time of the third visit that corresponded to a follow up or discharge visit (ACUTE/DISCHARGE). Percentage of positive samples is in bold text. The highest percentage corresponds to the assay condition with the best diagnostic read-out.

| **ADMISSION** | **NS1** | **ApoA1-cplx** | **ApoE-cplx** |
|---|---|---|---|
| Count Positive | 39 | 46 | 14 |
| Count Negative | 17 | 10 | 42 |
| Count NA | 0 | 0 | 0 |
| Average if NS1 Positive | 7503.7 | 1828.0 | 8724.4 |
| SD if NS1 Positive | 10161.5 | 1804.5 | 5963.6 |
| Average if NS1 Negative | | 593.2 | 10469.7 |
| SD if NS1 Negative | | 473.0 | 7396.6 |
| total count | 56 | 56 | 56 |
| % Positivity | **69.6** | **82.1** | **25.0** |

| **ACUTE/DISCHARGE** | NS1 | ApoA1-cplx | ApoE-cplx |
|---|---|---|---|
| Nb Positive | 23 | 20 | 28 |
| Nb Negative | 32 | 36 | 28 |
| NA | 1 | 0 | 0 |
| Average if NS1 Positive | 1924.9 | 646.0 | 14478.8 |
| SD if NS1 Positive | 3326.3 | 502.6 | 9618.4 |
| Average if NS1 Negative | | 460.0 | 14804.1 |
| SD if NS1 Negative | | 378.3 | 10335.1 |
| Total patient nb | 55 | 56 | 56 |
| % Positivity | **41.8** | **35.7** | **50.0** |

### Example 6: Quantification of viral or host factors in the plasma of DENV-infected patients - Correlation levels between clinical grades and virological variables

Statistical analyses were performed to highlight significant concentration changes of the NS1-ApoA1 and NS1-ApoE complexes in function of the different degrees of severity according to the 2009 classification **(****Fig. 8****).** To evaluate differences in median concentrations across the degrees of severity, Anova tests have been used and p-values reported in **Fig. 8****.** The p-values are associated to the test of equality between median concentrations of the two degrees of severity (i.e. a small p-value corresponds to a reject of the equality between the two degrees) **(****Fig. 8****).** The amount of the NS1-ApoA1 complex in plasma samples shows a significant level of correlation with disease severity (p=0.015) while the values for the NS1-ApoE complex have no predictive value (p=0.71) on the day of admission at hospital. Moreover, the concentrations of the NS1-ApoA1 complex also correlate significantly with plasma leakage intensity (p=0.005) **(****Fig. 11****).** Loss of plasma fluid is a critical criteria used to evaluate the severity of dengue disease.

### Example 7: Identification of NS1-HDL complexes for different flaviviruses

Purified preparations of NS1 proteins specified by other flaviviruses than DENV, including Japanese encephalitis, West Nile, Zika, yellow fever and tick-borne encephalitis viruses, were mixed with normal human plasma in order to assess whether the different recombinant proteins could interact with HDL. At the end of the incubation period, the presence of putative NS1-HDL complexes was tested using the same capture ELISA format as for the DENV NS1 protein associated to ApoA1. This approach was made possible due to the broad cross-reactivity of the DEN NS1 MAb 17A12, which allowed to detect and immobilize the NS1 proteins of the different flaviviruses.

In our experimental conditions, we were able to show the formation of NS1-HDL complexes for all mosquito-borne NS1 tested, although to various levels, while the NS1 protein from tick-borne encephalitis virus did not show any signal **(****Fig.9****).** These discrepancies may be due to a variability in the affinity of MAb 17A12 for the different NS1 proteins, that would modulate the proportion of complexes immobilized on the ELISA plate, or to variations in the intrinsic ability of the different flavivirus NS1 to bind to HDL. These data show that binding of NS1 to HDL is a common feature among flaviviruses.

### Example 8: Dengue NS1-specific monoclonal antibodies (MAb) can prevent NS1 binding to HDL

The inventors tested the ability of anti-NS1 MAbs to interfere with the binding of DENV NS1 to HDL. For this purpose, we incubated purified NS1 with each MAb at a 1 Ab:1 NS1 protomer molar ratio and measured the residual binding of the NS1 hexamer by BLI on immobilized HDL **(****Fig. 12****).** The capacity of the different anti-NS1 MAbs to inhibit the NS1-HDL interaction varied from the absence of inhibition, as observed for MAb 8G6, to a nearly complete inhibition with MAb 17A12, which displayed a signal that was comparable to the background noise generated by the different MAbs tested in the absence of NS1. MAb 1A11 was also a potent inhibitor while MAbs 6D2 and 4F7 only partially inhibited the complex formation **(****Fig. 12****).** The BLI signal generated by the NS1 protein bound to MAb 8G6 was higher than the NS1 protein alone due to the higher mass of the NS1-8G6 complexes captured by the immobilized HDL particles and detected on the sensor. All other MAbs bound to NS1 showed reduced signal intensities compared to NS1 alone, in accordance with partial to total inhibition.

### Conclusion

The inventors have demonstrated and report herein that NS1 hijacks endogenous ApoA1-positive HDL and ApoB-positive LDL lipoproteins particles during the acute phase of the dengue disease. The dengue virus NS1 binds preferentially to HDL particles. Binding of NS1 to HDL modifies their functional status and induces activation of a pro-inflammatory response in human macrophages. In the late clinical phase, the concentrations of NS1-ApoA1 complexes - representative of the NS1-HDL species - are reduced and NS1 rather seems to colonize a broad range of ApoE-positive lipoprotein particles. This suggests that the interaction of the NS1 protein with host lipoproteins may be a highly dynamic process that varies over time, as a mean for the virus to control the fate of the diverse species of lipoprotein particles and their related metabolic pathways or, at to some extent, for the host to eliminate intoxicated HDL particles.

Detection of the complexes formed between DENV NS1 and host lipoproteins has a better diagnostic value than the detection of NS1 itself and the quantification of their concentration in blood should be included in a panel of prognostic markers that helps to define at the time of admission at hospital which patient is at risk of developing a severe hemorrhagic fever and/or shock. In the absence of a specific therapy, elaborating a decision tree would have a great impact in the field to improve clinical management and reduce mortality rates.

The NS1-HDL complexes can form in *in vitro* reconstitution assays for all mosquito-borne NS1 proteins of different flaviviruses, including DENV, Japanese encephalitis virus, West Nile virus, Zika virus and yellow fever virus. This observation points to common functional roots among the NS1 proteins of the flavivirus genus. The NS1-HDL complexes are all likely to play an important role in viral pathogenesis and may show useful diagnostic and prognostic value for all flaviviroses.

### REFERENCES

1. Bhatt S, Gething PW, Brady OJ, Messina JP, Farlow AW, Moyes CL, Drake JM, Brownstein JS, Hoen AG, Sankoh O, Myers MF, George DB, Jaenisch T, Wint GR, Simmons CP, Scott TW, Farrar JJ, Hay SI. The global distribution and burden of dengue. Nature. 2013 Apr 25;496 (7446):504-7.
2. WHO 1997. Dengue haemorrhagic fever: diagnosis, treatment, prevention and control, Geneva, World Health Organization; 1997.
3. WHO 2009. Dengue. Guidelines for diagnosis, treatment, prevention and control, Geneva, World Health Organization; 2009, WHO/HTM/NTD/DEN/2009.
4. Bandyopadhyay S, Lum LCS, Kroeger A. Classifying dengue: a review of the difficulties in using the WHO case classification for dengue haemorrhagic fever. Trop Med Int Health. 2006 Aug 1;11(8):1238-55.
5. Deen JL, Harris E, Wills B, Balmaseda A, Hammond SN, Rocha C, et al. The WHO dengue classification and case definitions: time for a reassessment. Lancet. 2006 Jul 8;368:170-3.
6. Phuong CXT, Nhan NT, Kneen R, Thuy PTT, van Thien C, Nga NTT, et al. Clinical diagnosis and assessment of severity of confirmed dengue infections in Vietnamese children: is the world health organization classification system helpful? Am J Trop Med Hyg. 2004 Feb;70(2):172-9.
7. Rigau-Perez JG. Severe dengue: the need for new case definitions. Lancet Infect Dis. 2006 May;6:297-302.
8. Santamaria R, Martinez E, Kratochwill S, Soria C, Tan LH, Nuñez A, et al. Comparison and critical appraisal of dengue clinical guidelines and their use in Asia and Latin America. Int Health. 2009 Dec;1(2):133-40.
9. Alexander N, Balmaseda A, Coelho ICB, Dimaano E, Hien TT, Hung NT, et al. Multicentre prospective study on dengue classification in four South-east Asian and three Latin American countries. Trop Med Int Health. 2011 Aug 1;16(8):936-48.
10. Flamand, Marie, ; Salmon, Jerome, ; Rey, Felix, ; Gutsche, Irina, ; Ermonval, Myriam, ; Kayal, Samer WO2009106986 (A2) - 2009-09-03 Nonstructural Protein Ns1 As A Novel Therapeutic Target Against Flaviviruses: Use Of Inhibiting Molecules Interfering With Ns1 Maturation Or Biological Activity.
11. Guzman MG, Halstead SB, Artsob H, Buchy P, Farrar J, Gubler DJ, Hunsperger E, Kroeger A, Margolis HS, Martinez E, Nathan MB, Pelegrino JL, Simmons C, Yoksan S, Peeling RW. Dengue: a continuing global threat. Nat Rev Microbiol. 2010 Dec;8(12 Suppl):S7-16.
12. Alcon-LePoder S, Sivard P, Drouet MT, Talarmin A, Rice C, Flamand M. Secretion of flaviviral non-structural protein NS1: from diagnosis to pathogenesis. Novartis Found Symp. 2006;277:233-47; discussion 247-53. Review.
13. Rastogi M, Sharma N, Singh SK. Flavivirus NS1: a multifaceted enigmatic viral protein. Virol J. 2016 Jul 29;13:131.
14. De Silva AM, Rey FA, Young PR, Hilgenfeld R, Vasudevan SG. Viral Entry and NS1 as Potential Antiviral Drug Targets. Adv Exp Med Biol. 2018;1062:107-113.
15. Kumarasamy V, Chua SK, Hassan Z, Wahab AH, Chem YK, Mohamad M, Chua KB. Evaluating the sensitivity of a commercial dengue NS1 antigen-capture ELISA for early diagnosis of acute dengue virus infection. Singapore Med J. 2007 Jul;48(7):669-73.
16. Alcon S, Talarmin A, Debruyne M, Falconar A, Deubel V, Flamand M. Enzyme-linked immunosorbent assay specific to Dengue virus type 1 nonstructural protein NS1 reveals circulation of the antigen in the blood during the acute phase of disease in patients experiencing primary or secondary infections. J Clin Microbiol. 2002 Feb;40(2):376-81.
17. Falconar AK, Young PR. Production of dimer-specific and dengue virus group cross-reactive mouse monoclonal antibodies to the dengue 2 virus non-structural glycoprotein NS1. J Gen Virol. 1991 Apr;72 ( Pt 4):961-5.
18. Young PR, Hilditch PA, Bletchly C, Halloran W. An antigen capture enzyme-linked immunosorbent assay reveals high levels of the dengue virus protein NS1 in the sera of infected patients. J Clin Microbiol. 2000 Mar;38(3):1053-7.
19. Zhang M, Charles R, Tong H, Zhang L, Patel M, Wang F, Rames MJ, Ren A, Rye KA, Qiu X, Johns DG, Charles MA, Ren G. HDL surface lipids mediate CETP binding as revealed by electron microscopy and molecular dynamics simulation. Sci Rep. 2015 Mar 4;5:8741.
20. Modhiran N, Watterson D, Muller DA, Panetta AK, Sester DP, Liu L, Hume DA, Stacey KJ, Young PR. Dengue virus NS1 protein activates cells via Toll-like receptor 4 and disrupts endothelial cell monolayer integrity. Sci Transl Med. 2015 Sep 9;7(304):304ra142.
21. Marais AD. Apolipoprotein E in lipoprotein metabolism, health and cardiovascular disease. Pathology. 2019 Feb;51(2):165-176.
22. Andries AC, Duong V, Ly S, Cappelle J, Kim KS, Lorn Try P, Ros S, Ong S, Huy R, Horwood P, Flamand M, Sakuntabhai A, Tarantola A, Buchy P. Value of Routine Dengue Diagnostic Tests in Urine and Saliva Specimens. PLoS Negl Trop Dis. 2015 Sep 25;9(9):e0004100.
23. Scheres SH, Valle M, Nuñez R, Sorzano CO, Marabini R, Herman GT, Carazo JM. Maximum-likelihood multi-reference refinement for electron microscopy images. J Mol Biol. 2005 Apr 22;348(1):139-49.
24. Scheres SH. RELION: implementation of a Bayesian approach to cryo-EM structure determination.J Struct Biol. 2012 Dec;180(3):519-30

## Claims

1. An *in vitro* method for the diagnostic, prognostic or monitoring of a flaviviral infection or associated disease, comprising detecting the presence or level of a complex formed by a flaviviral non-structural protein 1 (NS1) and plasma high-density lipoprotein (HDL) particles in a biological sample obtained from a subject.

2. The in vitro method according to claim 1, comprising:
a. Contacting the biological sample with an antibody specific for the flaviviral NS1 or an antibody specific for plasma lipoproteins to form an immunoreaction product;
b. detecting the presence of the immunoreaction product, and
c. quantitating the complex formed by NS1 and plasma high-density lipoprotein (HDL) particles.

3. The in vitro method according to claim 2, which comprises:
a. Contacting the biological sample with an antibody specific for the flaviviral NS1 to form a first immunoreaction product;
b. Contacting said first immunoreaction product with an antibody specific for plasma lipoproteins to form a second immunoreaction product;
c. detecting the presence of the second immunoreaction product, and
d. quantitating the complex formed by NS1 and plasma high-density lipoprotein (HDL) particles.

4. The in vitro method according to any one of claims 2 to 3, wherein the quantity of the complex is determined by capture ELISA using the antibody specific for the flaviviral NS1 coated on a solid support and the antibody specific for plasma lipoproteins as detection antibody; and/or wherein a third antibody raised against antibody specific for plasma lipoproteins and conjugated to a suitable label is used for detecting the presence of the second immunoreaction product.

5. The in vitro method according to any one of claims 1 to 4, wherein the plasma high-density lipoprotein (HDL) particles are Apolipoprotein A1 (ApoA1) positive lipoprotein particles.

6. The in vitro method according to any one of claims 2 to 5, wherein the antibody specific for plasma lipoproteins is an antibody specific for Apolipoprotein A1 (ApoA1).

7. The in vitro prognostic method according to any one of claims 1 to 6, which is for the prognostic of a severe form of a flaviviral infection in a subject infected with a flavivirus, wherein the more the level of complex, the less the risk to develop severe form of flaviviral infection; preferably wherein the method is performed in a biological sample obtained during primary or acute infection; and/or preferably wherein the complex is formed by a flaviviral non-structural protein 1 (NS1) and plasma Apolipoprotein A1 (ApoA1) positive lipoprotein particles.

8. The in vitro monitoring method according to any one of claims 1 to 6, which is carried out on biological samples obtained from said subject at different times during the disease.

9. The in vitro diagnostic method according to any one of claims 1 to 6, wherein the presence of complex formed by flaviviral NS1 and plasma high-density lipoprotein (HDL) particles in said sample is indicative of a flaviviral infection; preferably wherein complex formed by flaviviral NS1 and HDL particles are quantitated, more preferably complex composed by flaviviral NS1 and ApoA1 positive lipoprotein particles; and/or preferably wherein the method is repeatedly carried out on a biological sample obtained from said subject to quantitate complex of flaviviral NS1-ApoA1 positive lipoprotein particles, and complex of flaviviral NS1-ApoE positive lipoprotein particles.

10. Use of the presence or level of a biomarker in a biological sample obtained from a subject for the diagnostic, prognostic or monitoring of a flaviviral infection or associated disease, wherein the biomarker comprises a complex formed by a flaviviral non-structural protein 1 (NS1) and plasma high-density lipoprotein (HDL) particles as defined in any one of claims 1 and 5.

11. A kit for the diagnostic, prognostic or monitoring of a flaviviral infection or associated disease in a biological sample obtained from a subject, comprising:
a. An antibody specific for the flaviviral NS1;
b. An antibody specific for plasma lipoproteins which is specific for HDL, preferably for ApoA1; and further comprising an antibody specific for ApoB and/or ApoE;
c. Means for detecting the production of an immunoreaction product between said two antibodies and the complex formed by the flaviviral NS1 and plasma lipoprotein particles.

12. **The** kit according to claim 11, the in vitro method according to any one of claims 1 to 9, the use of the biomarker according to claim 10, wherein the flaviviral NS1 is the NS1 from Dengue virus and/or the flaviviral infection is a Dengue virus infection.

13. **The** kit according to any one of claims 11 or 12, the in vitro method according to any one of claims 1 to 9, the use of the biomarker according to claim 10, wherein the biological sample is blood, plasma or serum and/or wherein the subject is human.

## Patentansprüche

1. *In-vitro*-Verfahren zur Diagnose, Prognose oder Überwachung einer Flavivirus-Infektion oder einer damit verbundenen Erkrankung, das das Nachweisen des Vorhandenseins oder des Spiegels eines Komplexes umfasst, der aus einem Flavivirus-Nichtstrukturprotein 1 (NS1) und High-Density-Lipoprotein(HDL)-Plasmapartikeln gebildet wird, in einer von einem Patienten entnommenen biologischen Probe.

2. In-vitro-Verfahren nach Anspruch 1, umfassend:
a. Kontaktieren der biologischen Probe mit einem Antikörper, der spezifisch für das Flavivirus-NS1 ist, oder einem Antikörper, der spezifisch für Plasmalipoproteine ist, um ein Immunreaktionsprodukt zu bilden;
b. Nachweisen des Vorhandenseins des Immunreaktionsprodukts, und
c. Quantifizieren des Komplexes, der durch NS1 und High-Density-Lipoprotein(HDL)-Plasmapartikel gebildet wird.

3. In-vitro-Verfahren nach Anspruch 2, das Folgendes umfasst:
a. Kontaktieren der biologischen Probe mit einem Antikörper, der spezifisch für das Flavivirus-NS1 ist, um ein erstes Immunreaktionsprodukt zu bilden;
b. Kontaktieren des ersten Immunreaktionsprodukts mit einem Antikörper, der spezifisch für Plasmalipoproteine ist, um ein zweites Immunreaktionsprodukt zu bilden;
c. Nachweisen des Vorhandenseins des zweiten Immunreaktionsprodukts, und
d. Quantifizieren des Komplexes, der durch NS1 und High-Density-Lipoprotein(HDL)-Plasmapartikel gebildet wird.

4. In-vitro-Verfahren nach einem der Ansprüche 2 bis 3, wobei die Menge des Komplexes mittels Capture-ELISA bestimmt wird, unter Verwendung des Antikörpers, der spezifisch für das Flavivirus-NS1 ist und auf einer festen Trägeroberfläche aufgebracht wird, und des Antikörpers, der spezifisch für Plasmalipoproteine ist, als Nachweisantikörper verwendet wird; und/oder wobei ein dritter Antikörper, der gegen den Antikörper spezifisch für Plasmalipoproteine gerichtet und mit einem geeigneten Marker konjugiert ist, zum Nachweisen des Vorhandenseins des zweiten Immunreaktionsprodukts verwendet wird.

5. In-vitro-Verfahren nach einem der Ansprüche 1 bis 4, wobei die High-Density-Lipoprotein(HDL)-Plasmapartikel Apolipoprotein A1 (ApoA1)-positive Lipoproteinpartikel sind.

6. In-vitro-Verfahren nach einem der Ansprüche 2 bis 5, wobei der Antikörper, der spezifisch für Plasmalipoproteine ist, ein Antikörper ist, der spezifisch für Apolipoprotein A1 (ApoA1) ist.

7. In-vitro-Prognoseverfahren nach einem der Ansprüche 1 bis 6, das für die Prognose einer schweren Form einer Flavivirus-Infektion bei einem mit einem Flavivirus infizierten Patienten bestimmt ist, wobei gilt: Je höher der Spiegel des Komplexes ist, desto geringer ist das Risiko, eine schwere Form der Flavivirus-Infektion zu entwickeln; wobei das Verfahren vorzugsweise an einer während der Primär- oder akuten Infektion entnommenen biologischen Probe durchgeführt wird; und/oder wobei der Komplex vorzugsweise aus einem Flavivirus-Nichtstrukturprotein 1 (NS1) und Plasma-Apolipoprotein A1 (ApoA1)-positiven Lipoproteinpartikeln gebildet wird.

8. In-vitro-Überwachungsverfahren nach einem der Ansprüche 1 bis 6, das an biologischen Proben durchgeführt wird, die von dem Patienten zu verschiedenen Zeitpunkten während der Erkrankung entnommen wurden.

9. In-vitro-Diagnoseverfahren nach einem der Ansprüche 1 bis 6, wobei das Vorhandensein eines Komplexes, der durch Flavivirus-NS1 und High-Density-Lipoprotein(HDL)-Plasmapartikel in der Probe gebildet wird, auf eine Flavivirus-Infektion hinweist; wobei der Komplex, der durch Flavivirus-NS1 und HDL-Partikel gebildet wird, vorzugsweise quantifiziert wird, noch besser der Komplex, der aus Flavivirus-NS1 und ApoA1-positiven Lipoproteinpartikeln besteht; und/oder wobei das Verfahren vorzugsweise wiederholt an einer biologischen Probe durchgeführt wird, die von dem Patienten entnommen wurde, um den Komplex aus Flavivirus-NS1-ApoA1-positiven Lipoproteinpartikeln und den Komplex aus Flavivirus-NS1-ApoE-positiven Lipoproteinpartikeln zu quantifizieren.

10. Verwendung des Vorhandenseins oder des Spiegels eines Biomarkers in einer biologischen Probe, die von einem Patienten entnommen wurde, zur Diagnose, Prognose oder Überwachung einer Flavivirus-Infektion oder einer damit verbundenen Erkrankung, wobei der Biomarker einen Komplex umfasst, der durch ein Flavivirus-Nichtstrukturprotein 1 (NS1) und High-Density-Lipoprotein(HDL)-Plasmapartikel gebildet wird, wie in einem der Ansprüche 1 und 5 definiert.

11. Kit für die Diagnose, Prognose oder Überwachung einer Flavivirus-Infektion oder einer damit verbundenen Erkrankung in einer biologischen Probe, die von einem Patienten entnommen wurde, umfassend:
a. einen Antikörper, der spezifisch für das Flavivirus-NS1 ist;
b. einen Antikörper, der spezifisch für Plasmalipoproteine ist, die spezifisch für HDL sind, vorzugsweise für ApoA1; und weiterhin einen Antikörper umfasst, der spezifisch für ApoB und/oder ApoE ist;
c. Mittel zum Nachweisen der Bildung eines Immunreaktionsprodukts zwischen den beiden Antikörpern und dem Komplex, der durch das Flavivirus-NS1 und Plasmalipoproteinpartikel gebildet wird.

12. Kit nach Anspruch 11, In-vitro-Verfahren nach einem der Ansprüche 1 bis 9, Verwendung des Biomarkers nach Anspruch 10, wobei das Flavivirus-NS1 das NS1 des Dengue-Virus ist und/oder die Flavivirus-Infektion eine Dengue-Virus-Infektion ist.

13. Kit nach einem der Ansprüche 11 oder 12, In-vitro-Verfahren nach einem der Ansprüche 1 bis 9, Verwendung des Biomarkers nach Anspruch 10, wobei die biologische Probe Blut, Plasma oder Serum ist und/oder wobei der Patient ein Mensch ist.

## Revendications

1. Méthode *in vitro* pour le diagnostic, le pronostic ou la surveillance d'une infection flavivirale ou d'une maladie associée, comprenant la détection de la présence ou du niveau d'un complexe formé par une protéine flavivirale non structurale 1 (NS1) et des particules de lipoprotéines plasmatiques de haute densité (HDL) dans un échantillon biologique obtenu auprès d'un sujet.

2. Méthode in vitro selon la revendication 1, comprenant :
a. la mise en contact de l'échantillon biologique avec un anticorps spécifique de la NS1 flavivirale ou un anticorps spécifique des lipoprotéines plasmatiques pour former un produit d'immunoréaction ;
b. la détection de la présence du produit d'immunoréaction, et
c. la quantification du complexe formé par la NS1 et des particules de lipoprotéines plasmatiques de haute densité (HDL).

3. Méthode in vitro selon la revendication 2, qui comprend :
a. la mise en contact de l'échantillon biologique avec un anticorps spécifique de la NS1 flavivirale pour former un produit d'immunoréaction ;
b. la mise en contact dudit premier produit d'immunoréaction avec un anticorps spécifique des lipoprotéines plasmatiques pour former un deuxième produit d'immunoréaction ;
c. la détection de la présence du deuxième produit d'immunoréaction, et
d. la quantification du complexe formé par la NS1 et des particules de lipoprotéine plasmatique de haute densité (HDL).

4. Méthode in vitro selon l'une quelconque des revendications 2 à 3, dans laquelle la quantité de complexe est déterminée par essai d'immunocapture, ELISA, à l'aide d'un support solide revêtu de l'anticorps spécifique de la NS1 flavivirale et de l'anticorps spécifique des lipoprotéines plasmatiques comme anticorps de détection ; et/ou dans laquelle un troisième anticorps dirigé contre l'anticorps spécifique des lipoprotéines plasmatiques et conjugué à un marqueur approprié est utilisé pour la détection de la présence du deuxième produit d'immunoréaction.

5. Méthode in vitro selon l'une quelconque des revendications 1 à 4, dans laquelle les particules de lipoprotéines plasmatiques de haute densité (HDL) sont des particules de lipoprotéines positives pour l'apolipoprotéine A1 (ApoA1).

6. Méthode in vitro selon l'une quelconque des revendications 2 à 5, dans laquelle l'anticorps spécifique des lipoprotéines plasmatiques est un anticorps spécifique de l'apolipoprotéine A1 (ApoA1).

7. Méthode de pronostic in vitro selon l'une quelconque des revendications 1 à 6, qui est destinée au pronostic d'une forme sévère d'une infection flavivirale chez un sujet infecté par un flavivirus, dans laquelle plus le niveau de complexes est élevé, moins le risque de développer une forme sévère d'infection flavivirale est élevé ; de préférence dans laquelle la méthode est réalisée dans un échantillon biologique obtenu au cours d'une infection primaire ou aiguë ; et/ou de préférence dans laquelle le complexe est formé par une protéine flavivirale non structurale 1 (NS1) et des particules de lipoprotéines plasmatiques positives pour l'apolipoprotéine A1 (ApoA1).

8. Méthode de surveillance in vitro selon l'une quelconque des revendications 1 à 6, qui est effectuée sur des échantillons biologiques obtenus auprès dudit sujet à différents moments au cours de la maladie.

9. Méthode de diagnostic in vitro selon l'une quelconque des revendications 1 à 6, dans laquelle la présence de complexes formés par une NS1 flavivirale et des particules de lipoprotéines plasmatiques de haute densité (HDL) dans ledit échantillon est indicative d'une infection flavivirale ; de préférence dans laquelle les complexes formés par une NS1 flavivirale et des particules de HDL sont quantifiés, plus préférentiellement les complexes composés d'une NS1 flavivirale et de particules de lipoprotéines positives pour ApoA1 ; et/ou de préférence dans laquelle la méthode est effectuée de manière répétée sur un échantillon biologique obtenu auprès dudit sujet pour quantifier des complexes de NS1 flavivirale-particules de lipoprotéines positives pour ApoA1, et des complexes de NS1 flavivirale-particules de lipoprotéines positives pour ApoE.

10. Utilisation de la présence ou du niveau d'un biomarqueur dans un échantillon biologique obtenu auprès d'un sujet pour le diagnostic, le pronostic ou la surveillance d'une infection flavivirale ou d'une maladie associée, dans laquelle le biomarqueur comprend un complexe formé par une protéine non structurale 1 (NS1) flavivirale et des particules de lipoprotéines plasmatiques de haute densité (HDL) tel que défini selon l'une quelconque des revendications 1 et 5.

11. Kit pour le diagnostic, le pronostic ou la surveillance d'une infection flavivirale ou d'une maladie associée dans un échantillon biologique obtenu auprès d'un sujet, comprenant :
a. un anticorps spécifique de la NS1 flavivirale ;
b. un anticorps spécifique des lipoprotéines plasmatiques qui est spécifique de HDL, de préférence de ApoA1 ; et comprenant en outre un anticorps spécifique de ApoB et/ou ApoE ;
c. des moyens de détection de la production d'un produit d'immunoréaction entre lesdits deux anticorps et le complexe formé par la NS1 flavivirale et les particules de lipoprotéines plasmatiques.

12. Kit selon la revendication 11, méthode in vitro selon l'une quelconque des revendications 1 à 9, utilisation du biomarqueur selon la revendication 10, dans lequel ou laquelle la NS1 flavivirale est la NS1 issue du virus de la Dengue et/ou l'infection flavivirale est une infection par le virus de la Dengue.

13. Kit selon l'une quelconque des revendications 11 ou 12, méthode in vitro selon l'une quelconque des revendications 1 à 9, utilisation du biomarqueur selon la revendication 10, dans lequel ou laquelle l'échantillon biologique est du sang, du plasma ou du sérum et/ou dans lequel ou laquelle le sujet est humain.
